# EUROPÄISCHE PATENTSCHRIFT

(11) **EP 1 282 639 B1**
(45) Veröffentlichungstag und Bekanntmachung des Hinweises auf die Patenterteilung: **17.08.2005**
(21) Anmeldenummer: 01919443.0
(22) Anmeldetag: 07.04.2001
(51) Int. Cl.: C07K 14/00, C07H 21/00, C12Q 1/68, A61K 38/04, C07F 9/40

(54) **PEPTIDNUKLEINSAURE-DERIVATE MIT NEGATIVER LADUNG, MITTEL UND VERFAHREN ZU IHRER HERSTELLUNG**
POLYAMIDE NUCLEIC ACID DERIVATIVES, AGENTS AND METHODS FOR PRODUCING THE SAME
POLYAMIDNUKLEINSAURE-DERIVATE, MITTEL UND VERFAHREN ZU IHRER HERSTELLUNG

(30) Priorität: 18.04.2000 DE 10019136
(43) Veröffentlichungstag der Anmeldung: 12.02.2003
(73) Patentinhaber: Aventis Pharma Deutschland GmbH, 65929 Frankfurt am Main (DE)
(72) Erfinder: UHLMANN, Eugen, 61479 Glashütten (DE); BREIPOHL, Gerhard, 60529 Frankfurt (DE); WILL, David, William, 65830 Kriftel (DE)
(86) Internationale Anmeldenummer: PCT/EP2001/004027
(87) Internationale Veröffentlichungsnummer: WO 2001/079249

(56) Entgegenhaltungen:
- WO-A-96/11205
- WO-A-99/33867
- DE-A- 19 508 923
- DE-A- 19 640 974

## Beschreibung

Die vorliegende Erfindung bezieht sich auf N-terminal phosphorylierte Polyamidnukleinsäure-(PNA)-Derivate mit verbesserten Eigenschaften, deren Verwendung, sowie Mittel und Verfahren zu ihrer Herstelung.

Polyamidnukleinsäuren, auch Peptidnukleinsäure (PNA) genannt, binden mit höherer Affinität als natürliche Oligonukleotide an komplementäre Zielsequenzen (DNA oder RNA) und haben gegenüber natürlicher DNA zudem den Vorteil, dass sie im Serum sehr stabil sind. PNA waren ursprünglich beschrieben als unnatürliche Nukleinsäure-Analoga, in denen das gesamte Zucker-Phosphat-Rückgrat durch N-(2-Aminoethyl)-glycin-Einheiten ersetzt ist (M. Egholm et al. (1991) Science 254, 1497-1500; WO 92/20702; M. Egholm et al. Nature (1993) 365, 566-568; P. Nielsen, (1994) Bioconjugate Chem. 5, 3-7; E. Uhlmann et al. (1998) Angewandte Chemie Int. Ed. Engl. 37, 2796-2823). Als Basen werden die in der Nukleotidchemie üblichen natürlich vorkommenden oder auch nicht natürlich vorkommenden Nukleobasen oder deren Prodrugform benutzt, also Vorstufen, die erst im Organismus durch Biotransformation in die freie Base überführt werden. Darüberhinaus wurden PNAs beschrieben, in denen nicht alle Positionen des Rückgrates Basenreste tragen (Greiner et al. (1999) Helv. Chim Acta 82, 2151), und in denen Aminoethylglycin durch komplexere Einheiten ersetzt ist (Uhlmann et al. (1998) Angewandte Chem. Int. Ed. 37, 2796; Falkiewicz (1999) Biochim. Pol., 46, 509-529). In der deutschen Patentanmeldung DE 195 08 923 A1 werden zudem PNAs beschrieben, die sich durch eine hohe Stabilität auszeichnen und in der Lage sind, die Zell- und Nukleusmembran zu passieren.

Der ladungsneutrale Charakter des PNA-Rückgrats ist ein wichtiges Merkmal dieser Substanzklasse mit weitreichenden Konsequenzen. Es wird dem neutralen Charakter der PNA und der damit verbundenen verringerten Ladungsabstoßung zugeschrieben, dass PNA selbst bei niedriger Salzkonzentration an komplementäre DNA und RNA bindet (z.B. Peptide Nucleic Acids: Protocols and Applications; Peter E. Nielsen und Michael Egholm (Edit.) Horizon Scientific Press, 1999, 3), wobei die Watson-Crick-Basenpaarungsregeln befolgt werden. Daher kann PNA im Prinzip für zahlreiche Anwendungen herangezogen werden, in denen sonst natürliche Oligonucleotide bzw. Oligonucleotid-Derivate eingesetzt werden. Darüber hinaus ergeben sich aber aufgrund der einzigartigen Bindungseigenschaften auch zahlreiche Anwendungen, die mit natürlichen Oligonucleotiden nicht möglich sind (siehe zum Beispiel: Peptide Nucleic Acids: Protocols and Applications; Peter E. Nielsen und Michael Egholm (Edit.) Horizon Scientific Press, 1999). Beispielsweise ist mit PNA eine Stranginvasion an doppelsträngiger DNA beobachtet worden.

Typische Beispiele zur Anwendung von PNA beinhalten deren Verwendung zur Inhibition der Genexpression durch sequenzspezifische Bindung an die zelluläre DNA oder RNA. "Antisense-Agenzien" sind kurze einzelsträngige Nukleinsäure-Derivate, die über Watson-Crick Basenpaarung an eine komplementäre mRNA binden, deren Übersetzung in das entsprechende Protein gehemmt werden soll (Uhlmann und Peyman (1990) Chem. Rev. 90, 543; Larsen et al. (1999) Biochem. Biophys. Acta 1489, 159). "Anti-Gen-Agenzien" binden über Hoogsteen-Basenpaarung in die große Furche der DNA-Doppelhelix unter Ausbildung einer Tripelhelix, wodurch die Transkription der Gene sequenzspezifisch gehemmt wird (Praseuth et al. (1999) Biochem. Biophys. Acta 1489, 181). Die Genexpression kann auch durch sogenannte "Decoy"-Oligomere, die die Bindungsregionen für Transkriptionsfaktoren nachahmen, spezifisch gehemmt werden. Durch die Behandlung mit Decoy-Agenzien lassen sich bestimmte Transkriptionsfaktoren sequenzspezifisch abfangen und dadurch eine Aktivierung der Transkription verhindern (Mischiati et al. (1999) J. Biol. Chem. 274, 33114). Eine weitere Gruppe von intrazellulär wirkenden Oligonucleotid-Derivaten, die Chimeraplasten, werden zur gezielten Genkorrektur herangezogen (Cole-Strauss et al. (1996) Science 273, 1386-1389).

PNA können daher als Arzneimittel und/oder Diagnostikum bzw. zur Herstellung von Arzneimitteln und/oder Diagnostika verwendet werden.

Für diagnostische Zwecke und in der Molekularbiologie kann PNA beipielsweise nach Markierung mit Biotin, Fluorescein oder anderen Labeln als spezifische Hybridisierungssonde eingesetzt werden. Für die Einführung der Markergruppen sind in der Literatur bislang vier Methoden beschrieben (Oerum et al. (1999), in Peptide Nucleic Acids: Protocols and Applications, Seiten 81-86; Lohse et al. (1997) Bioconjugate Chem. 8, 503). Die erste Methode beruht auf der Markierung der freien (entschützten) PNA nach deren Synthese in Lösung. Dabei wird der Aminoterminus der PNA mit einer aktivierten Carbonsäure oder einem Isothiocyanat umgesetzt. Oft werden aber zusätzliche Lysin-Reste in die PNA eingeführt, die dann mit Fluoresceinisothiocyanat (FITC) umgesetzt werden.

Bei der zweiten Methode wird die geschützte PNA noch an der Festphase am Aminoterminus mit aktivierten Carbonsäure-Derivaten oder Isothicyanaten modifiziert. Diese Methode eignet sich nur für Markergruppen, die unter den Bedingungen der Entschützung der PNA und während der Abspaltung vom Träger stabil sind. Als reaktive Reagenzien werden in beiden Fällen bevorzugt Isothiocyanate (P. Wittung et al., (1995) FEBS Lett. 375, 27) oder aktivierte Carbonsäuren, wie beispielsweise N-Hydroxysuccinimidester (NHS), eingesetzt (Oerum et al., 1999). Ein Nachteil der Reaktion mit den NHS-Derivaten ist, dass sie oft nur in schlechten Ausbeuten gelingt. Daher wird häufig 8-Amino-3,6-dioxaoctansäure als Linker bzw. Spacer zwischen PNA und Markergruppe kondensiert (Oerum et al., 1999). Beide Verknüpfungen erfolgen über Amidbindungen bzw. Thioharnstoffbindungen, die als solche aber eher zu Unlöslichkeit führen. Alternativ werden die Carbonsäuren mit Hilfe von in der Peptid-Chemie üblichen Aktivatoren, wie beispielsweise HBTU, TBTU oder HATU zur Reaktion gebracht.

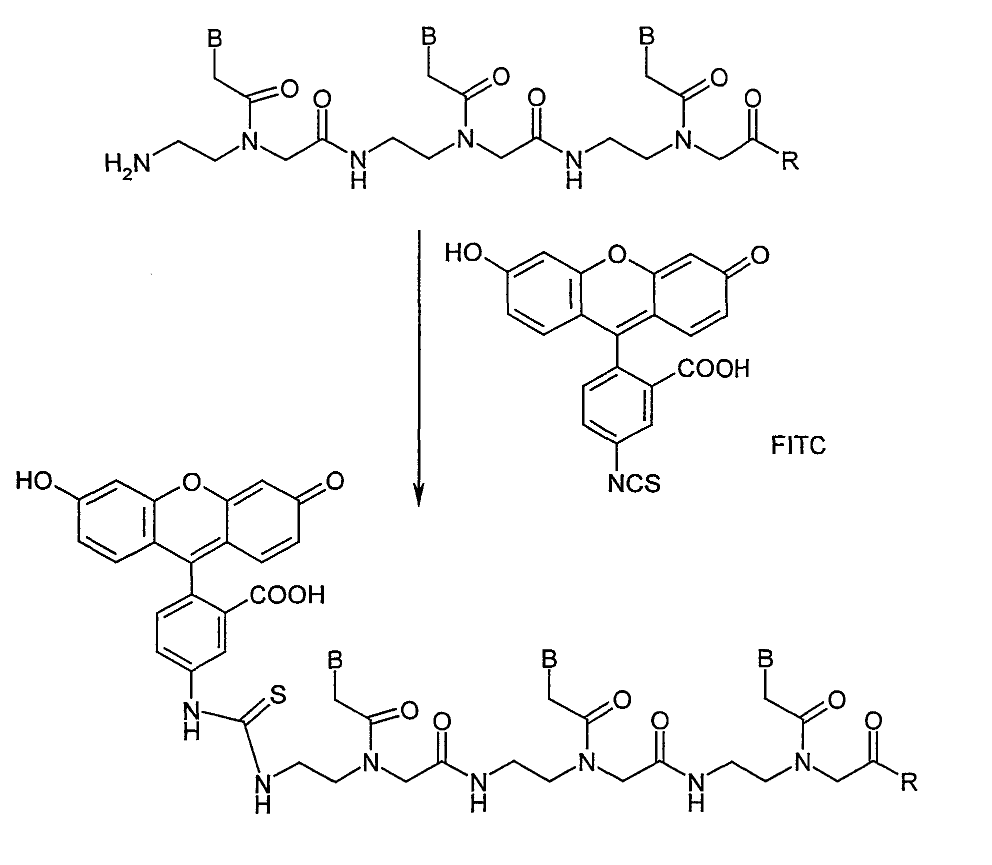

Bei einer dritten Methode werden Fluorescein-konjugierte Monomere bei der Festphasensynthese von PNA verwendet, wobei die Fluoreszenz-Markierung über eine Amidbindung erfolgt (Lohse et al. (1997) Bioconjugate Chem. 8, 503), die wiederum zu relativ schwerlöslichen Konjugaten führt.

Eine vierte Methode verwendet PNA-Peptid-Konjugate, in denen der Peptid-Teil ein Substrat für eine Protein-Kinase bildet (Koch et al. (1995) Tetrahedron Lett. 36, 6933). Auf diese Weise wird also nicht der PNA-Teil modifiziert, sondern der Serin-Rest des Peptid-Segments wird enzymatisch phosphoryliert. Mit dieser Methode kann also nur radioaktives Phosphat, aber beispielsweise kein Fluorescein oder Biotin in das Peptid-Segment des PNA-Peptid-Konjugats eingeführt werden.

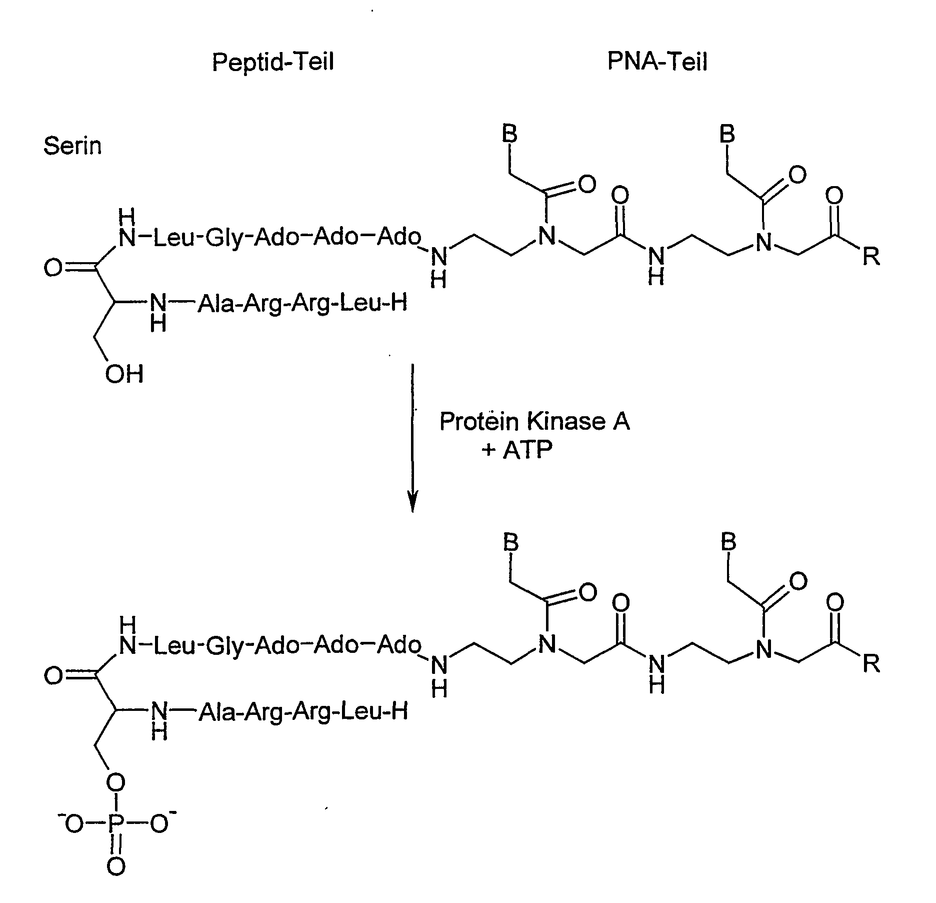

Es ist bekannt, dass PNA in wässriger Lösung, also auch unter physiologischen Bedingungen, zur Aggregation neigt. PNA ist daher in wässrigem Puffer schlecht löslich und steht dann nicht für die Hybridisierung an komplementäre Sequenzen zur Verfügung. PNA zeigt zudem eine hohe Affinität zu verschiedenen Materialien wie Sephadex® (Fa. Pharmacia), Bond Elut® (Fa. Varian), oder verschiedene HPLC-Chromatographiematerialien, die bei der Aufreinigung der Oligomeren Verwendung finden, so daß die PNA oft nur in schlechten Ausbeuten zu isolieren ist. Daher ist es notwendig, die PNA mit Lysin oder anderen positiv geladenen Aminosäuren (über den C-Terminus) zu konjugieren (Egholm et al (1992) J. Am. Chem. Soc. 114, 1895). Guaninreiche PNA-Sequenzen tendieren ganz besonders zur Aggregation, weshalb von der Verwendung solcher PNA abgeraten wird (siehe "Guidelines for sequence design of PNA oligomers" in Peptide Nucleic Acids: Protocols and Applications (1999) Seiten 253-255). Besonders schwerlöslich sind beispielsweise längere Fluorescein-markierte PNA-Oligomere, wobei die Zugabe eines organischen Lösemittels und Erwärmen auf 50°C empfohlen wird.

Die Reinigung der schwerlöslichen lipophilen PNA-Derivate ist besonders schwierig. Bei der HPLC werden oft mehrere Peaks detektiert, die auf PNA-Aggregate zurückzuführen sind. Die für die Reinigung und Trennung von Nukleinsäuren häufig angewandte Technik der Polyacrylamid (PAA)-Gelelektrophorese ist für diese PNA-Derivate nicht möglich.

Bei den oben beschriebenen Methoden der Derivatisierung von PNA wird die Marker-Gruppe stets durch Knüpfung einer Amidbindung oder Thioamidbindung eingeführt, wobei relativ schwerlösliche PNA-Derivate gebildet werden. Insbesondere bei der Einführung lipophiler Reste, wie beispielsweise des Fluoresceins, entstehen schwerlösliche PNA-Derivate. Da die Markierungs-Reaktionen zudem oft in schlechten Ausbeuten verlaufen, besteht eine zu lösende Aufgabe darin, neue PNA-Derivate bereitzustellen, welche in hohen Ausbeuten herzustellen sein sollen und vorteilhaften Eigenschaften aufweisen, wie verbesserter Löslichkeit, verbessertem Bindungsverhalten, besserer zellulärer Aufnahme aufweisen sollen, und die zudem den Einsatz effektiver Reinigungsmethoden für die PNA-Oligomeren erlaubt.

Die Aufgabe wird erfindungsgemäß gelöst durch die Bereitstellung von PNA-Derivaten, die am N-Terminus des PNA-Rückgrates einen oder mehrere Phosphoryl-Reste tragen, wobei neben Oxo- auch Thio- und Imino-Derivate umfasst sind, und wobei mindestens einer der Phosphoryl-Reste eine oder mehrere deprotonierbare Gruppen, vorzugsweise Hydroxy- oder MercaptoGruppen trägt. Die Phosphoryl-Reste sind über eine Sauerstoff-Phosphor, Schwefel-Phosphor oder eine Stickstoff-Phosphor-Bindung entweder direkt oder über einen Spacer mit dem PNA-Rückgrat verknüpft, wobei der Spacer beispielsweise ein Alkanoylamid, ein Poly(alkoxy)carboxamid oder eine Aminosäure sein kann, die gegebenfalls am α- oder β-C-Atom eine Seitenkette tragen, wobei diese Seitenkette keinen Phosphoryl-Rest tragen darf. Beispiele für Phosphoryl-Reste sind Phosphat, Phosphonat, Thiophosphat, Phosphoamidat oder substitierte Phosphoryl-Reste, wobei substituierte Phosphoryl-Reste gegebenenfalls eine oder mehrere Markergruppen, oder Gruppen zur Quervernetzung, oder Gruppen, die die intrazelluläre Aufnahme begünstigen, oder Gruppen, die die Bindungsaffinität des PNA-Derivats an Nukleinsäuren steigern, tragen

Unter Markergruppen (Labeln) versteht man dabei Gruppen, die eine qualitative oder quantitative Bewertung der chemischen oder biologischen Aktivität der PNA-Derivate erlauben, beispielsweise Biotin oder Fluoreszein. Unter Quervemetzung (Cross-Linking) versteht man die Ausbildung intra- bzw. intermolekularer Bindungen zwischen räumlich benachbarten Funktionalitäten. Eine Gruppe zur Quervemetzung ist beispielsweise die Psoralengruppe.

Die Erfindung betrifft vorzugsweise PNA-Derivate der Formel I, 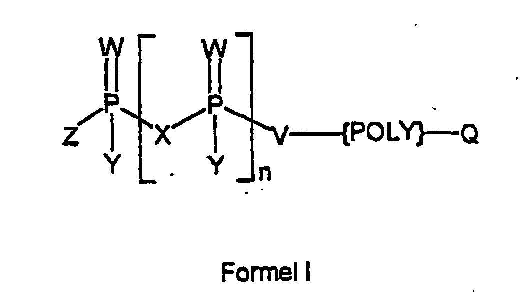 wobei
- V: gleich Sauerstoff, Schwefel, NR₁, eine Gruppe U-(CR₃R₄)_{u'}-C(O)-NH oder eine Gruppe U-(CH₂CH₂O)_{u'}-CH₂-C(O)-NH ist,
- U: unabhängig voneinander gleich Sauerstoff, Schwefel oder NH ist,
- u': unabhängig voneinander gleich 1 bis 10 ist, vorzugsweise 1 bis 4, besonders bevorzugt 1,
- W: unabhängig voneinander gleich Sauerstoff, Schwefel oder NR₁ ist,
- Y: unabhängig voneinander gleich Hydroxy, Mercapto, Oxyanion, Thioat oder NR₁R₂ ist,
- R₁ und R₂: unabhängig voneinander einen Rest bestehend aus Wasserstoff oder C₁-C₆-Alkyl bedeuten, vorzugsweise Wasserstoff,
- R₃ und R₄: unabhängig voneinander einen Rest bestehend aus Wasserstoff oder C₁-C₆-Alkyl oder den Rest einer Aminosäure-Seitenkette bedeuten, vorzugsweise Wasserstoff,
- X: unabhängig voneinander gleich eine Gruppe U-(C₂-C₂₂-Alkandiyl)-U oder eine Gruppe U-(CH₂CH₂-O)_{u'} ist,
oder gleich eine Markergruppe, oder eine Gruppe zur Quervernetzung, oder eine Gruppe, die die intrazelluläre Aufnahme begünstigt, oder eine Gruppe, die die Bindungsaffinität des PNA-Derivats an Nukleinsäuren steigert, ist, beispielsweise ein bifunktioneller Fluorescein-, Rhodamin-, TAMRA-, Biotin-, Pyren-, Dinitrophenyl-, Cholesteryl-, Acridin-, Adamantyl-, Vitamin E-, Cyaninfarbstoff-, Dabcyl-, Edans-, Lexitropsin-, Psoralen-, BODIPY-, ROX-, R6G- oder Digoxigenin-Rest,
- Z: gleich Hydroxy, Mercapto, Oxyanion, Thioat oder NR₁R₂, C₁-C₂₂-Alkyl, C₁-C₈-Arylalkyl, C₁-C₂₂-Alkyl-U, C₁-C₈-Arylalkyl-U, Hydroxy-C₁-C₁₈-U, Aminoalkyl-U, Mercaptoalkyl-U ist,
oder eine Gruppe der Formel R₉(CH₂CH₂-O)ₘ ist, wobei Rg gleich Hydroxy, Amino oder C₁-C₂₂-Alkoxy und m gleich 1 bis 100 ist, vorzugsweise 2 bis 10,
oder gleich eine Markergruppe, oder eine Gruppe zur Quervernetzung, oder eine Gruppe, die die intrazelluläre Aufnahme begünstigt, oder eine Gruppe, die die Bindungsaffinität des PNA-Derivats an Nukleinsäuren steigert, ist, beispielsweise ein monofunktioneller oder bifunktioneller Fluorescein-, Rhodamin-, TAMRA-, Biotin-, Pyren-, Dinitrophenyl-, Cholesteryl-, Acridin-, Adamantyl-, Vitamin E-, Cyaninfarbstoff-, Dabcyl-, Edans-, Lexitropsin-, Psoralen-, BODIPY-, ROX-, R6G- oder Digoxigenin-Rest,
- n: gleich 0 bis 10 ist, vorzugsweise 0 bis 3,
- Q: gleich Hydroxy, Amino, NHR₇, NR₇R₈, Aminosäure-Derivat oder ein Peptid-Rest ist,
- R₇ und R₈: unabhängig voneinander C₁-C₁₈-Alkyl oder Hydroxy-C₁-C₁₈-Alkyl sind,
mit der Maßgabe, dass mindestens ein Rest Y oder Z gleich Hydroxy, Mercapto, Oxyanion oder Thioat ist.
{POLY} wird beschrieben durch die Formel II.

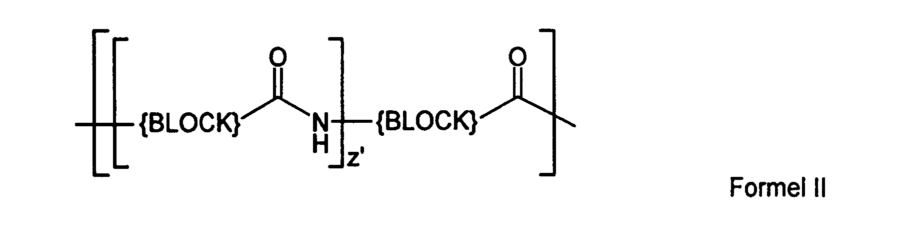

Das PNA-Rückgrat ist damit aus z'+1 Monomeren aufgebaut, wobei {BLOCK} unabhängig voneinander eine Gruppe ist ausgewählt aus Formel IIIA, 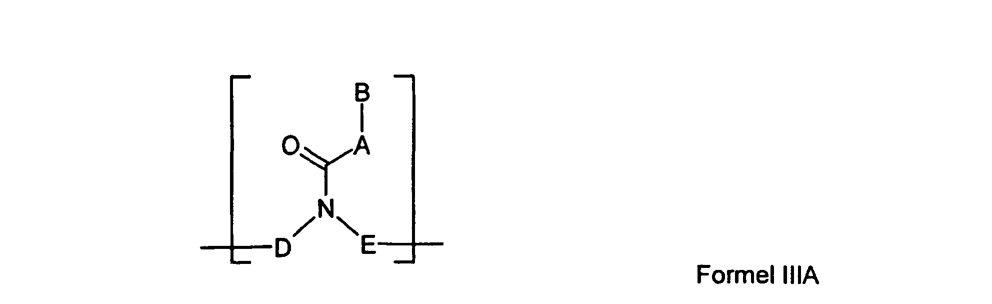 oder aus Formel IIIB (Greiner et al. (1999) Helv. Chim Acta 82, 2151), 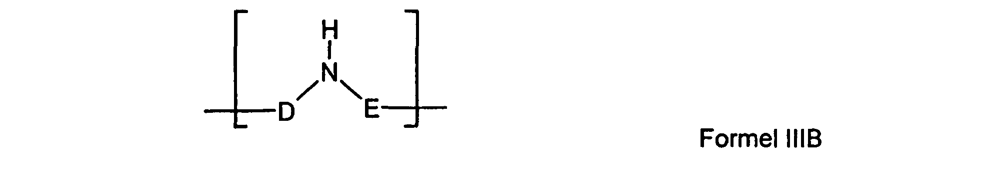 oder aus den Formeln IV A bis IV G (Uhlmann et al. (1998) Angewandte Chem. Int. Ed. 37, 2796; Falkiewicz (1999) Biochim. Pol., 46, 509-529), 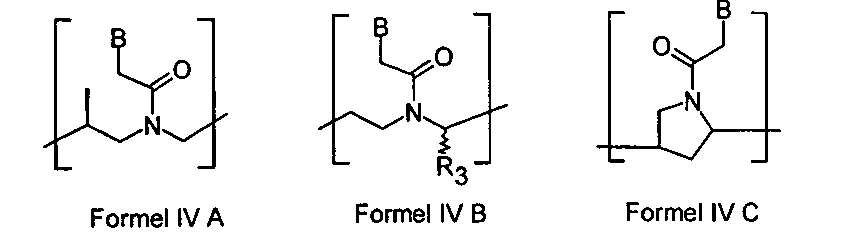 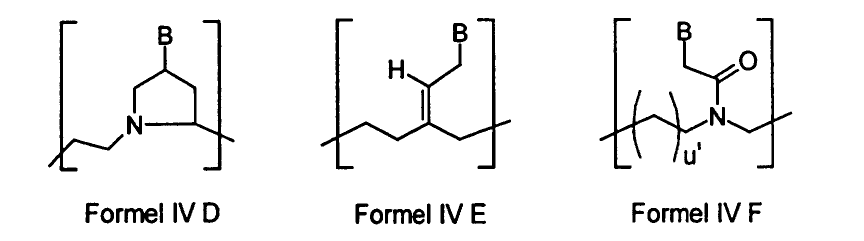 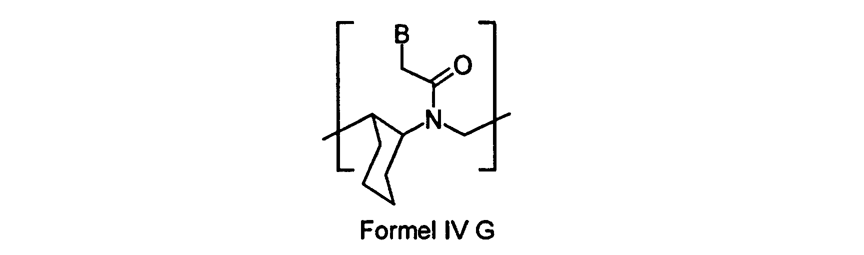 wobei jeder Baustein {BLOCK} verschieden sein kann,
und wobei
- A: unabhängig voneinander eine Gruppe (CR₁R₂)ₛ ist, wobei s gleich 1 bis 3 ist, vorzugsweise 1,
- B: unabhängig voneinander entweder ein aromatischer Rest, der auch heteroaromatischen Charakter besitzen kann, oder Wasserstoff, oder Hydroxy oder C₁-C₁₈-Alkyl ist,
oder eine in der Nucleotidchemie übliche natürlich vorkommende oder eine nicht natürlich vorkommende Nucleobase oder deren Prodrugform ist,
wobei mindestens ein Rest B in Formel II eine Nucleobase ist,
- D: unabhängig voneinander eine Gruppe (CR₃R₄)ₜ ist, wobei t gleich 2 bis 10 ist, vorzugsweise 2 bis 4, besonders bevorzugt 2, wobei R₃ und R₄ die oben genannte Bedeutung haben, und zwei benachbarte Reste D auch einen C₅-C₈-Cycloalkylring bilden können,
- E: unabhängig voneinander eine Gruppe (CR₅R₆)_{u'} ist, wobei zwei benachbarte Reste R₅ und R₆ auch einen C₅- bis C₈-Cycloalkyl-Ring oder eine Spiroverbindung bilden können,
- R₅ und R₆: unabhängig voneinander einen Rest bestehend aus Wasserstoff oder C₁-C₆-Alkyl sind, vorzugsweise Wasserstoff, oder den Rest einer Aminosäure-Seitenkette bedeuten,
- z': gleich 0 bis 100 ist, vorzugsweise 1-20, besonders bevorzugt 4-15.

Die Erfindung betrifft darüberhinaus physiologisch verträglichen Salze der PNA-Derivate der Formel I. Physiologisch verträglichen Salze sind u.a. beschrieben in Remingtons Pharmaceutical Science (1985) Mack Publishing Company, Easton, PA, USA, Seite 1418. Bevorzugt sind Ammoniumsalze, Trialkylammoniumsalze, Alkalimetallsalze (wie Natrium- und Kaliumsalze) und Erdalkalisalze (wie Magnesium- und Calciumsalze). Besonders bevorzugt sind Natriumsalze.

Überraschenderweise wurde gefunden, dass bereits eine negative Teilladung am Phosphoryl-Rest ausreicht, um die Eigenschaften der Verbindungen der Formel I entscheidend zu verbessern. Während PNA selbst bei der PAA-Gelelektrophorese nicht wandert, wandern die Verbindungen der Formel I zur Anode. Da der Phosphoryl-Rest während der Synthese der Verbindungen der Formel I erst im letzten Zyklus eingeführt wird, wandern bei der PAA-Gelelektrophorese nur die erfindungsgemäßen Verbindungen im elektrischen Feld, während alle Fehlsequenzen und Nebenprodukte nicht wandern und somit auf extrem einfache Art abgetrennt werden können.

Die Hydroxy- bzw. Mercapto-Substituenten der Phosphoryl-Reste der efindungsgemässen PNA-Derivate können in einem pH-Bereich von 4,5 bis 14, vorzugsweise 6,5 bis 12, besonders bevorzugt 6,5 bis 9 deprotoniert werden. Die Eigenschaft der Ionisierbarkeit der Phosphoryl-Reste kann vorteilhaft zur Reinigung der Verbindungen der Formel I ausgenutzt werden. Einerseits können die Verbindungen der Formel I durch Elektrophorese, beispielsweise Polyacrylamid-Gelelektrophorese, gereinigt werden. Andererseits ist eine Reinigung mit Hilfe von Anionenaustauschern möglich. Dabei werden die gewünschten Produkte bevorzugt durch Anwendung eines Salzgradienten, beispielsweise eines Kochsalz-Gradienten, oder eines pH-Gradienten eluiert. Besonders einfach und effizient lassen sich die erfindungsgemäßen PNA-Derivate der Formel I über Anionenaustauscher reinigen. Es zeigte sich, dass die ungeladenen Nebenprodukte nicht auf dem Anionenaustauscher retardiert werden, während das geladene Produkt auf der Säule haftete. Nach Waschen mit Wasser konnte das gewünschte Produkt mit Essigsäure oder einer Kochsalzlösung in reiner Form isoliert werden. Als Anionenaustauscher verwendet man bevorzugt starke Anionenaustauscher, oder Mixed-Mode-Phasen, wie beispielsweise ®Oasis MAX (Waters GmbH, Eschborn).

Weiterhin zeigte sich, dass die erfindungsgemäßen Verbindungen der Formel I generell besser in wässrigem Medium löslich sind als die entsprechenden PNA-Oligomeren ohne den Phosphoryl-Rest. Dies macht sich ganz besonders bei den lipophilen Derivaten, wie beispielsweise den Fluorescein-Derivaten oder Hexadecyl-Derivaten, in Form einer stark verbesserten Löslichkeit in wässrigem Medium bemerkbar.

Als weiterer überraschender positiver Effekt zeigte sich, dass die Einführung eines Phosphoryl-Restes beispielsweise als Phosphat oder auch in Form einer lipophilen Derivatisierung (z. B. als Hexadecylphosphodiester) die Affinität der PNA an komplementäre DNA oder RNA erhöht. Dieser Effekt war nicht zu erwarten, da die starke Binding von PNA an komplementäre DNA oder RNA dem neutralen Charakter der PNA und der damit verbundenen reduzierten Ladungsabstossung zugeschrieben wurde (z.B. Peptide Nucleic Acids: Protocols and Applications; Peter E. Nielsen und Michael Egholm (Edit.) Horizon Scientific Press, 1999, 3).

Besonders effektiv erfolgte die Einführung des Biotins über einen Phosphoryl-Rest. Die biotinylierte PNA der Formel I (X und/oder Z = Biotinrest) zeigten als Hybridisierungssonden bessere Bindungseigenschaften und weniger störende unspezifische Hintergrund-Effekte als entsprechende biotinylierte DNA-Sonden.

Im Gegensatz zur ungeladenen PNA können die erfindungsgemäßen PNA-Derivate der Formel I auch im elektrischen Feld wandern, wodurch eine Mikrolokalisierung und eine Konzentrierung an immobilisierten komplementären Nukleinsäurederivaten möglich ist. Für die polyanionischen Oligonucleotide wurde diese Methode zur raschen Bestimmung von Basenmisspaarungen mit Hilfe des elektrischen Felds bereits beschrieben (Sosnowski et al. (1997) Proc. Natl. Acad. Sci. U.S.A. 94, 1119).

Die Erfindung betrifft besonders PNA-Derivate, in denen A und E gleich CH₂ sind. Weiterhin betrifft die Erfindung besonders PNA-Derivate, in denen D gleich (CH₂)₂ sind. Bevorzugt sind ferner PNA-Derivate der Formel I, in denen V, W und Y gleich Sauerstoff sind.

Beispiele für natürliche Basen sind Adenin, Cytosin, 5-Methylcytosin, Guanin, Thymin und Uracil. Beispiele für unnatürliche Basen sind Purin, 2,6-Diaminopurin, N⁴N⁴-Ethanocytosin, N⁶N⁶-Ethano-2,6-diaminopurin, 5-(C₃-C₆)-Alkinyl-uracil, 5-(C₃-C₆)-Alkinyl-cytosin, 5-(1-Propargylamino)-uracil, 5-(1-Propargylamino)-cytosin, Phenoxazin, 9-Aminoethoxyphenoxazin, 5-Fluor-uracil oder Pseudoisocytosin, 5-(Hydroxymethyl)uracil, 5-Aminouracil, Pseudouracil, Dihydrouracil, 5-(C₁-C₆)-Alkyl-uracil, 5-(C₁-C₆)-Alkyl-cytosin, 5-(C₂-C₆)-Alkenyl-cytosin, 5-Fluorcytosin, 5-Chloruracil, 5-Chlorcytosin, 5-Bromuracil, 5-Bromcytosin, 7-Deazaadenin, 7-Deazaguanin, 8-Azapurin, oder 7-Deaza-7-substituierte Purine.

Q ist bevorzugt ein Hydroxyalkylamino-Rest, insbesondere ein Hydroxyhexylamino-Rest, oder ein Derivat einer bekannten natürlichen oder unnatürlichen Aminosäure oder eines Peptids. Als Peptidsequenzen kommen bevorzugt solche in Betracht, die Organverteilung oder die zelluläre Lokalisierung des PNA optimieren, wie beispielsweise Transportan, Insulin-like Growth Factor, Nuclear Localisation Signale oder andere Carriersequenzen (Larsen et al. (1999) Biochim. Biophys. Acta 159-166). Das Peptid kann auch als Affinitäts-Tag dienen, wie etwa eine (His)₆ Kette.

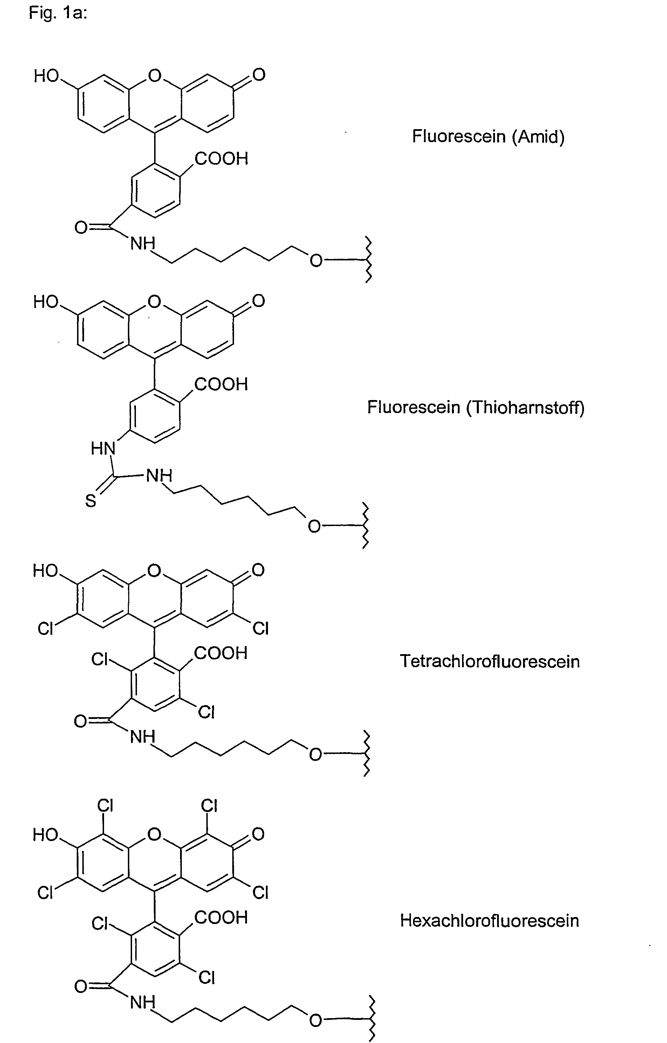

Die vorliegende Erfindung ermöglicht eine breite Variation der Reste X und Z (Beispiele sind in Figuren 1a, 1b, 2a, 2b, 3a und 3b gegeben) und erlaubt so die Einführung unterschiedlicher spezifischer Funktionsmerkmale in PNA.

Eine bevorzugte Ausführungsform von Z ist ein C₁- bis C₂₂-Alkyl-Rest. Bevorzugt sind auch C₁- bis C₂₂-Alkoxy-Reste, insbesondere C₁₆-Alkoxy-Reste. Andere bevorzugte Reste sind Hydroxy-(C₁-C₁₈-Alkoxy)-Reste, insbesondere HO(CH₂)₃₋₁₂O. Weiterhin bevorzugt sind Aminoalkoxy-Reste, insbesondere 6-Aminohexoxy- und 5-Aminopentoxy-Reste. Weiterhin bevorzugt sind Reste der Formel R₉(CH₂CH₂-O)ₘ, wobei R₉ gleich Hydroxy, Amino oder C₁-C₂₂-Alkoxy ist, bevorzugt aber Hydroxy ist, und m gleich 0 bis 100, bevorzugt 2 bis 10 ist. Besonders bevorzugt sind HO(CH₂CH₂-O)₂, HO(CH₂CH₂-O)₆ und H₂N-(CH₂CH₂-O)₂. Weitere bevorzugte Beispiele von Z umfassen Mercaptoalkoxy-Reste, insbesondere 6-Mercaptohexyloxy.

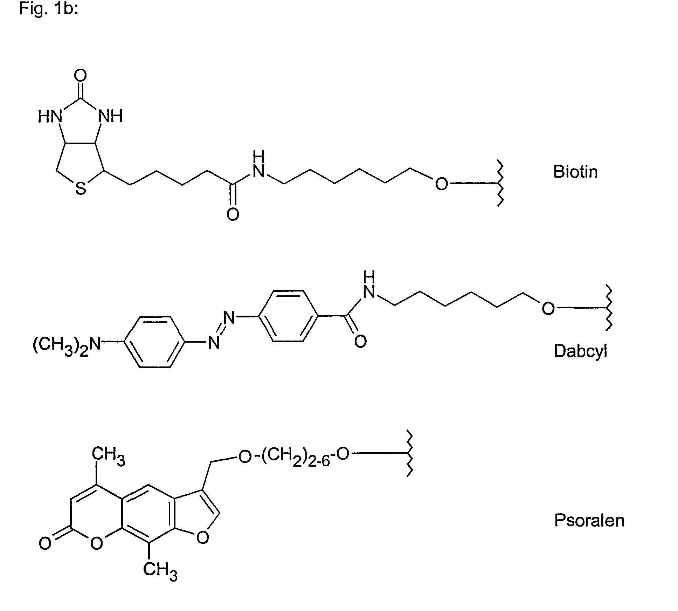

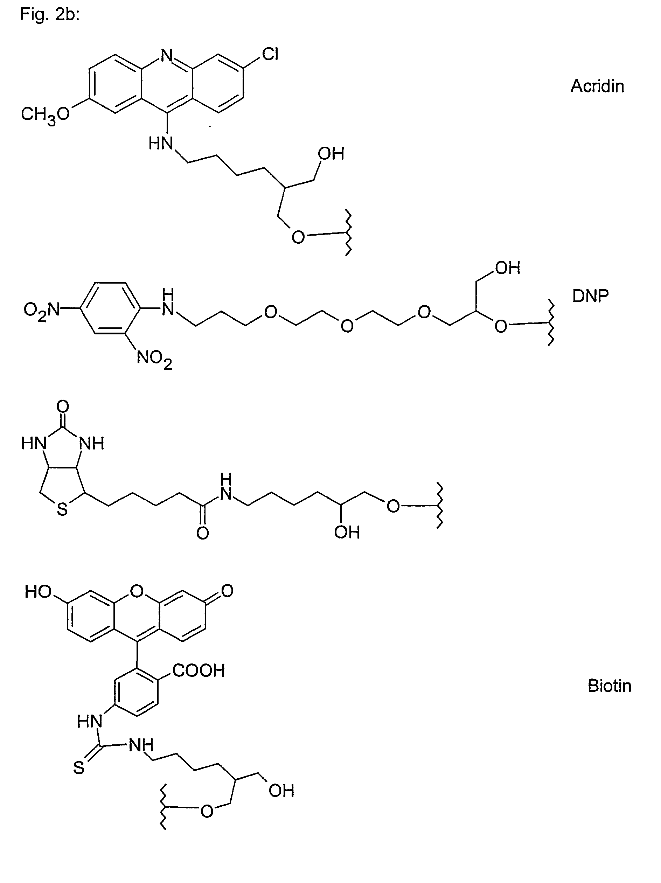

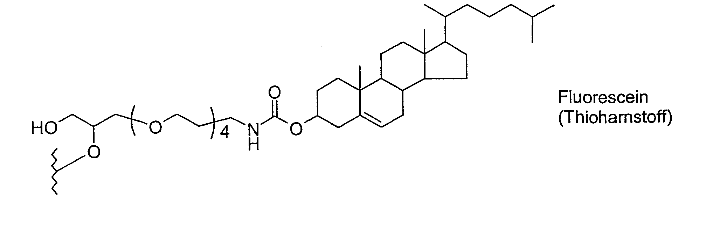

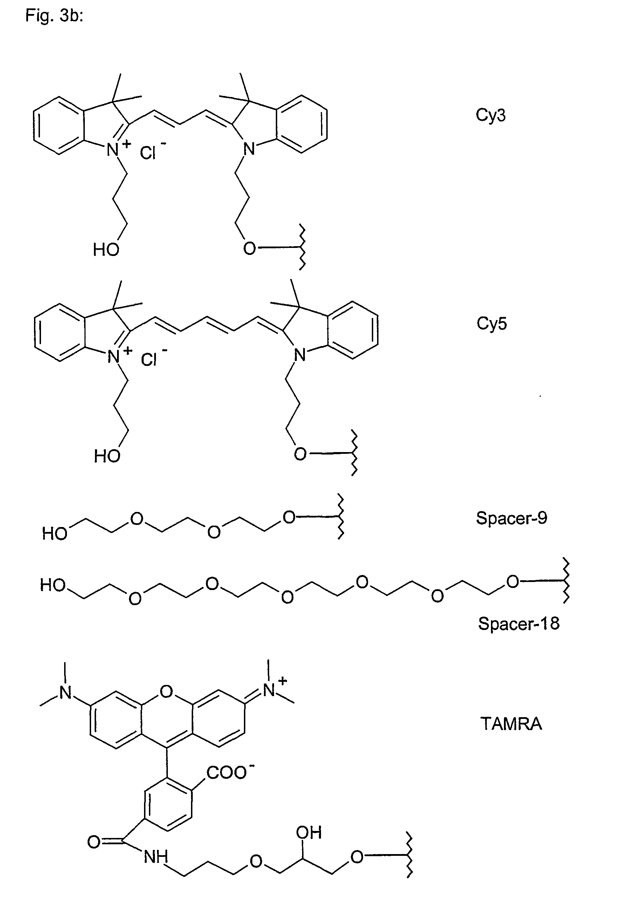

In einer weiteren bevorzugten Ausführungsform umfasst Z eine fluoreszierende Gruppe wie Fluorescein, Rhodamin, TAMRA oder einen Cyanin-Farbstoff. Bevorzugte fluoreszierende Gruppen sind in den Figuren 1a bis 3b zu finden. Ganz besonders bevorzugt ist auch Biotin für Z. Andere bevorzugte Gruppen für Z umfassen Dabcyl, Psoralen, Acridin, DNP und Cholesterol (Figuren 1b und 2b), BODIPY-, ROX- oder R6G-Reste (Su-Chun Hung et al. (1998) Analytical Biochemistry 255, 32-38) und Digoxigenin (Tarrason et al., Methods in Enzyology (1999) Vol. 313, 257-268). Darüberhinaus kann Z gleich einer Gruppe bestehend aus einem monofunktionellen oder einem bifunktionellen Fluorescein-, Rhodamin-, TAMRA-, Biotin-, Pyren-, Dinitrophenyl-, Cholesteryl-, Acridin-, Adamantyl-, Vitamin-E-, Cyaninfarbstoff-, Dabcyl-, Edans-, Lexitropsin-, oder Psoralen-Rest sein. Monofunktionelle Endgruppen sind beispielhaft in den Figuren 1a, 1b, 2a und 3a ausgeführt, bifunktionelle verbrückende Gruppen sind in Figuren 2b und 3b aufgeführt. In einer anderen bevorzugten Ausführungsform ist n gleich 0, d.h. der PNA-Teil trägt nur einen Phosphat- oder Phosphorylrest.

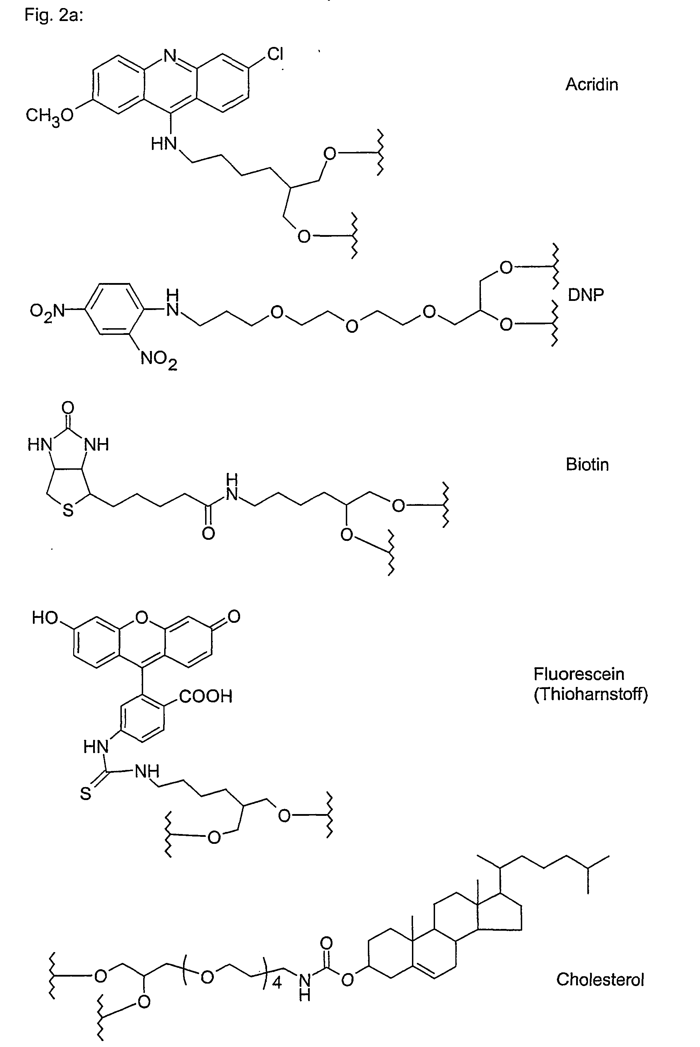

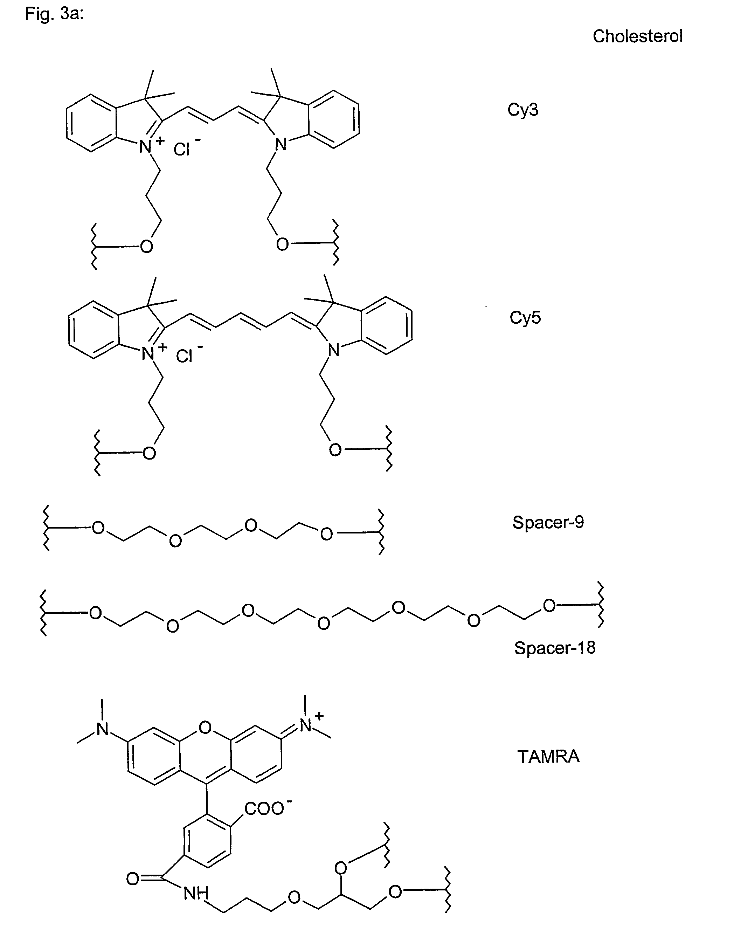

Eine bevorzugte Ausführungsform von X ist U-(C₂-C₂₂-Alkandiyl)-U, insbesondere O-(C₂-C₂₂-Alkandiyl)-O, besonders bevorzugt O-(CH₂)₂₋₆-O. Eine andere bevorzugte Ausführungsform von X ist eine Gruppe der Formel U-(CH₂CH₂-O)_{u'} ist, wobei u' gleich 1 bis 10 ist, bevorzugt 1 bis 6, und wobei U bevorzugt Oxy ist. In einer weiteren bevorzugten Ausführungsform umfasst X eine fluoreszierende Gruppe wie Fluorescein, Rhodamin, TAMRA oder einen Cyanin-Farbstoff, beispielsweise Cy3® (Fa. Amersham Pharmacia Biotech). Bevorzugte bifunktionelle Gruppen sind in den Figuren 2a und 3a zu finden. Ganz besonders bevorzugt ist auch Biotin für X. Andere bevorzugte Gruppen für X umfassen Dabcyl-, Psoralen-, Acridin-, DNP-, Cholesterol-, BODIPY-, Digoxigenin-, ROX- und R6G-Reste.

Die unterschiedlichen Reste für X und Z in Formel I können unterschiedliche Funktionen erfüllen. Die Fluorescein-Reste haben weitreichende Anwendungen bei der DNA-Sequenzierung, Signalamplifikation oder als Marker zur Bestimmung der zellulären Aufnahme von PNA. Die Cyanin-Farbstoff-Reste (Cy3® und Cy5®) ergeben ein wesentlich intensiveres und länger anhaltendes Fluoreszenzsignal als Fluorescein selbst. Der Psoralen-Rest wird zur Quervernetzung (Cross-Linking) mit komplementären Nukleinsäuren eingesetzt. Der Acridin-Rest ist ein effektiver Interkalator und kann damit die Bindungsaffinität der PNA verstärken. Biotin-, Acridin- und Psoralen-Derivate können auch für Antisense-Experimente eingesetzt werden. Weiterhin können Hexadecyloxy- und Cholesterol-Derivate zur Erhöhung der Membrangängigkeit der PNA angewandt werden. DNP-markierte Verbindungen der Formel I lassen sich mit anti-DNP-Antikörpern nachweisen. Aminoalkoxy-Reste lassen sich zur Kupplung weiterer Gruppen, wie beispielsweise Lexitropsin (vgl. Beispiel 17; PNA-16), nutzen. In ähnlicher Weise lassen sich auch Mercaptoalkoxy-Gruppen zur weiteren Derivatisierung nutzen.

Die Erfindung betrifft weiterhin die Verwendung der PNA-Derivate der Formel I zur Berutstellung von Anzneimitteln. Diese Arzneimittel können zur Prävention und/oder Behandlung von Krankheiten eingesetzt werden, die mit der Expression bzw. einer Überexpression bestimmter Gene einhergehen. Weiterhin betrifft die Erfindung die Verwendung von PNA-Derivaten der Formel I als Diagnostikum. Diese Diagnostika können zur In-Vitro Früherkennung der oben genannten Krankheiten eingesetzt werden.

Als Arzneimittel bzw. Diagnostikum lassen sich die PNA-Derivate der Formel I in Abhängigkeit ihrer Sequenz als Antisense-, Antigene-, Decoy-, und Chimeraplast-Agenzien nutzen.
Die erfindungsgemäßen PNA-Derivate werden insbesondere zur Herstellung von Arzneimitteln zur Behandlung von Krankheiten verwendet, bei denen definierte Gene durch Überexpression ursächlich bzw. beteiligt ist.

Diese Arzneimittel können beispielsweise zur Behandlung von Erkrankungen verwendet werden, die durch Viren hervorgerufen werden, beispielsweise durch CMV, HIV, HSV-1, HSV-2, Influenza, VSV, Hepatitis B oder Papilloma Viren, wobei die entsprechende Virus-Sequenz das Target ist.

Erfindungsgemäße Antisense-PNA-Derivate, die gegen solche Targets wirksam sind, haben beispielsweise folgende Basensequenz.
a) gegen CMV, z. B.
b) gegen HIV, z. B.
c) gegen HSV-1, z.B.

Solche Arzneimittel eignen sich beispielsweise auch zur Behandlung von Krebs. Vorzugsweise können dabei Sequenzen zum Einsatz kommen, die gegen Targets gerichtet sind, die für Krebsentstehung bzw. Krebswachstum verantwortlich sind, insbesondere durch die Inhibition der Telomerase (E. Matthes et al. (1999) Nucleic Acids Res. 27, 1152). Solche Targets sind weiterhin:
1) Nucleare Onkoproteine wie beispielsweise c-myc, N-myc, c-myb, c-fos, c-fos/jun, PCNA, p120,
2) Cytoplasmische/Membran-assoziierte Onkoproteine wie beispielsweise EJras, c-Ha-ras, N-ras, rrg, bcl-2, cdc-2, c-raf-1, c-mos, c-src, c-abl, c-ets,
3) Zelluläre Rezeptoren wie beispielsweise EGF-Rezeptor, Her-2, c-erbA, VEGF-Rezeptor (KDR-1), Retinoid-Rezeptoren, Protein-Kinase regulatorische Untereinheit, c-fms, Tie-2, c-raf-1 Kinase, PKC-alpha, Protein Kinase A (R1 alpha),
4) Cytokine, Wachstumsfaktoren, Extrazelluläre Matrix wie beispielsweise CSF-1, IL-6, IL-1a, IL-1b, IL-2, IL-4, IL-6, IL-8, bFGF, VEGF, Myeloblastin, Fibronectin.

Antisense-PNA-Derivate, die gegen solche Targets wirksam sind, haben beispielsweise folgende Basen-Sequenz:
a) gegen c-Ha-ras, z. B.
b) bFGF, z.B.
c) c-myc, z.B.
d) c-myb, z.B.
e) c-fos, z.B.
f) p120, z. B.
g) EGF-Rezeptor, z.B.
h) p53 Tumorsuppressor, z.B.
i) bcl-2, z. B.
k) VEGF, z. B.
I) c-raf Kinase, z. B.
m) PKC-alpha, z. B.
n) Protein Kinase A, z. B.

Arzneimittel enthaltend PNA-Derivate der Formel I eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.
Antisense-PNA-Derivate, die gegen solche Targets wirksam sind, haben beispielsweise folgende Basen-Sequenz:
a) VLA-4, z. B.
b) ICAM-1, z. B.
c) ELAM-1, z. B.
d) Integrin alpha(V), z. B.

Arzneimittel enthaltend PNA-Derivate der Formel I eignen sich beispielsweise auch zur Verhinderung der Restenose. Dabei können PNA-Sequenzen zum Einsatz kommen, die gegen Targets gerichtet sind, die für Proliferation oder Migration verantwortlich sind. Solche Targets sind beispielsweise:
1) Nucleare Transaktivator-Proteine und Cycline wie beispielsweise c-myc, c-myb, c-fos, c-fos/jun, Cycline und cdc2-Kinase
2) Mitogene oder Wachstumsfaktoren wie beispielsweise PDGF, bFGF, VEGF, EGF, HB-EGF und TGF-β.
3) Zelluläre Rezeptoren wie beispielsweise bFGF-Rezeptor, EGF-Rezeptor und PDGF-Rezeptor.

Antisense-PNA-Derivate, die gegen solche Targets wirksam sind, haben beispielsweise folgende Basen-Sequenz:
a) c-myb, z. B.
b) c-myc, z. B.
c) cdc2-Kinase, z. B.
d) PCNA (proliferating cell nuclear antigen of rat), z. B.

PNA-Derivate können ebenfalls zur Behandlung von Vitiligo und anderen Depigmentierungskrankheiten bzw. Depigmentierungsstörungen (z.B. der Haut, Haare, Augen) wie beispielsweise Albinismus und Psoriasis verwendet werden, oder zur Behandlung von Asthma, wobei die Expression des Adenosin-A1-Rezeptors, Adenosin-A3-Rezeptors, Bradikinin-Rezeptors oder von IL-13 mit Hilfe geeigneter Antisense Agenzien inhibiert werden. Eine solche Basensequenz ist beispielsweise:

Arzneimittel enthaltend ein PNA-Derivat der Formel I können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.
Bevorzugte Verabreichungsformen sind topische Applikationen, lokale Applikationen wie beispielsweise mit Hilfe eines Katheters oder durch Inhalation, Injektionen bzw. Infusionen und die orale Verabreichung. Zur Injektion werden die PNA-Derivate der Formel I in einer flüssigen Lösung, vorzugsweise in einem physiologisch annehmbaren Puffer, wie z.B. Hank's Lösung oder Ringer's Lösung, formuliert. Die Oligonucleotide können aber auch in fester Form formuliert werden und vor dem Gebrauch gelöst oder suspendiert werden. Die für die systematische Verabreichung bevorzugten Dosierungen betragen ca. 0,01 mg/kg bis ca. 50 mg/kg Körpergewicht und Tag.

Die Erfindung betrifft weiterhin pharmazeutische Zubereitungen, die PNA-Derivate der Formel I und/oder deren physiologisch verträgliche Salze neben pharmazeutisch einwandfreien Träger- und/oder Zusatzstoffen enthalten. Die PNA-Derivate der Formel I und/oder deren physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine topische, perkutane, parenterale oder enterale Anwendung gestatten und die als aktiven Bestandteil eine wirksame Dosis mindestens eines PNA-Derivats, neben üblichen pharmazeutisch einwandfreien Träger- und Zusatzstoffen enthalten. Die Zubereitungen enthalten normalerweise etwa 0,1 bis 90 Gew.-% der therapeutisch wirksamen Verbindung. Zur Behandlung von Hautkrankheiten wird eine topische Anwendung, z.B. in Form von Salben, Lotionen oder Tinkturen, Emulsionen, Suspensionen bevorzugt.

Die Herstellung der pharmazeutischen Präparate erfolgt in an sich bekannter Weise, (z. B. Remingtons Pharmaceutical Sciences, Mack Publ. Co., Easton, PA.), wobei pharmazeutisch inerte anorganische und/oder organische Trägerstoffe verwendet werden. Für die Herstellung von Pillen, Tabletten, Dragees und Hartgelatinekapseln kann man z.B. Lactose, Maisstärke und/oder Derivate derselben, Talk, Stearinsäure und/oder deren Salze etc. verwenden. Trägerstoffe für Weichgelatinekapseln und/oder Suppositorien sind z.B. Fette, Wachse, halbfeste und flüssige Polyole, natürliche und/oder gehärtete Öle etc. Als Trägerstoffe für die Herstellung von Lösungen und/oder Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate und/oder Rods eignen sich Mischpolymerisate aus Glykolsäure und Milchsäure. Darüber hinaus sind Liposomenformulierungen, die dem Fachmann bekannt sind, geeignet (N. Weiner, Drug Develop Ind Pharm 15 (1989) 1523; "Liposome Dermatics, Springer Verlag 1992), beispielsweise HVJ-Liposomen (Hayashi, Gene Therapy 3 (1996) 878). Die dermale Applikation kann beispielsweise auch auch unter Zuhilfenahme ionophoretischer oder Methoden und/oder mit Hilfe der Elektroporation erfolgen.

Eine pharmazeutische Zubereitung kann neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel und/oder Lösungsvermittler und/oder Mittel zur Erzielung eines Depoteffekts, sowie Salze zur Veränderung des osmotischen Drucks, Überzugsmittel und/oder Antioxidantien enthalten. Sie können auch zwei oder mehrere verschiedene PNA-Derivate der Formel I und/oder deren physiologisch verträgliche Salze enthalten sowie ferner neben mindestens einem PNA-Derivate der Formel I einen oder mehrere andere therapeutisch wirksame Stoffe. Die Dosis kann innerhalb weiter Grenzen variieren und ist in jedem einzelnen Fall den individuellen Gegebenheiten anzupassen.

Weiterhin betrifft die Erfindung die Verwendung von PNA-Derivaten der Formel I als Diagnostikum, insbesondere als Hilfsmittel in der DNA-Diagnostik und in der Molekularbiologie (siehe zum Beispiel: Peptide Nucleic Acids: Protocols and Applications; Peter E. Nielsen und Michael Egholm (Edit.) Horizon Scientific Press, 1999). In der DNA-Diagnostik spielen Gensonden, auch DNA-Sonden oder Hybridization-Sonden genannt, eine große Rolle zum sequenzspezifischen Nachweis bestimmter Gene. Eine Gensonde besteht im allgemeinen aus einer Erkennungssequenz und einer bzw. mehreren geeigneten Markergruppen (Labeln). Die Spezifität der Bestimmung einer Targetsequenz in einer Analysenprobe mittels Hybridisierung mit einer komplementären Gensonde wird durch die Erkennungssequenz und deren chemische Struktur determiniert. Diese Technik kann auf PNA übertragen werden. Die PNA hat gegenüber den Oligonucleotiden natürlicher Struktur den Vorteil, dass sie eine höhere Affinität zur Targetsequenz und eine höhere Fähigkeit zur Basendiskriminierung aufweist.

Die Anwendung der Verbindungen der Formeln I bezieht sich daher auch auf die in-situ-Hybridisierung und Fluoreszenz-in-situ-Hybridisierung (FISH). Die in-situ-Hybridisierung kann beispielsweise auch zum Nachweis von Mikroorganismen und Viren dienen (Just et al. (1998) J. Vir. Method. 73, 163-174). Eine weitere Anwendung der erfindungsgemäßen Verbindungen bezieht sich zum Nachweis und Quantifizierung von Nukleinsäuren. Hierzu bedient man sich bevorzugt auch der Array-Technologie (Strother et al. J. Am. Chem. Soc. (2000) 122, 1205-1209; Niemeyer et al., Angew. Chem. (1999) 111, 3039-3043; Pirrung (1997) Chem. Rev. 97, 473-488), die einen hohen Probendurchsatz und hohe Empfindlichkeit aufweist. Dabei werden die PNA-Sonden auf einem geeigneten Träger oder PNA-Chip fixiert. Hierzu kann PNA wie in den Beispielen beschrieben synthetisiert werden und nachfolgend auf den Träger oder PNA-Chip fixiert werden. Alternativ kann die PNA direkt auf dem Träger hergestellt werden. Eine weitere Anwendung ist der Einsatz der Verbindungen der Formel I als Biosensoren zur Detektion von Nukleinsäuren (Wang et al (1996) J. Am. Chem. Soc. 118, 7667). Der Einsatz von PNA der Formel I mit einem Affinitätslabel, wie beispielsweise Histidyl-PNA, ist eine weitere Anwendung zur Reinigung von Nukleinsäuren (Oerum et al. (1999), in Peptide Nucleic Acids: Protocols and Applications).

Die Synthese des PNA-Rückgrates erfolgt nach den in der Literatur beschriebenen Methoden, zum Beispiel nach der tert-Butyloxycarbonyl- (BOC), 9-Fluorenylmethoxycarbonyl- (Fmoc) oder Monomethoxytrityl- (Mmt) Schutzgruppen-Strategie (Peptide Nucleic Acids: Protocols and Applications; Peter E. Nielsen und Michael Egholm (Edit.) Horizon Scientific Press, 1999). Vorzugsweise wird die Mmt-Schutzgruppe zum temporären Schutz der Aminofunktion des Aminoethylglycins verwendet und basenlabile Schutzgruppen an den heterozyklischen Nucleobasen (D. Will et al. (1995) Tetrahedron 51, 12069; Breipohl et al. (1997) Tetrahedron 53, 14671-14686). Beispiele für Monomerbausteine sind Verbindungen der Formel V bis V B, wobei A, B, D, E, u' und U obige Bedeutung haben. PG ist eine AminoSchutzgruppe wie beispielsweise Benzoyl, Anisoyl-, Isobutyroyl-, Acetyl-, tert-Butylbenzoyl (Breipohl et al. (1997) Tetrahedron 53, 14671-14686). TR ist eine säurelabile Schutzgruppe wie Dimethoxytrityl (Dmt) (für U = O und S) oder Mmt (für U = NH).

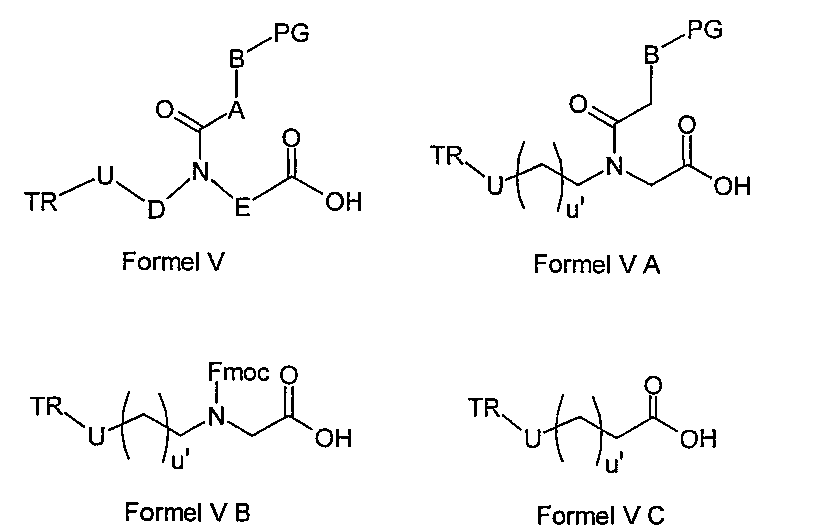

Nach Aufbau des PNA-Rückgrates kann die freie Aminofunktion des N-Terminus direkt mit einem entsprechenden Phosphorylierungsreagenz beispielsweise zum entsprechenden Phosphoramidat (U = NR₁ in Formel I) umgesetzt werden.

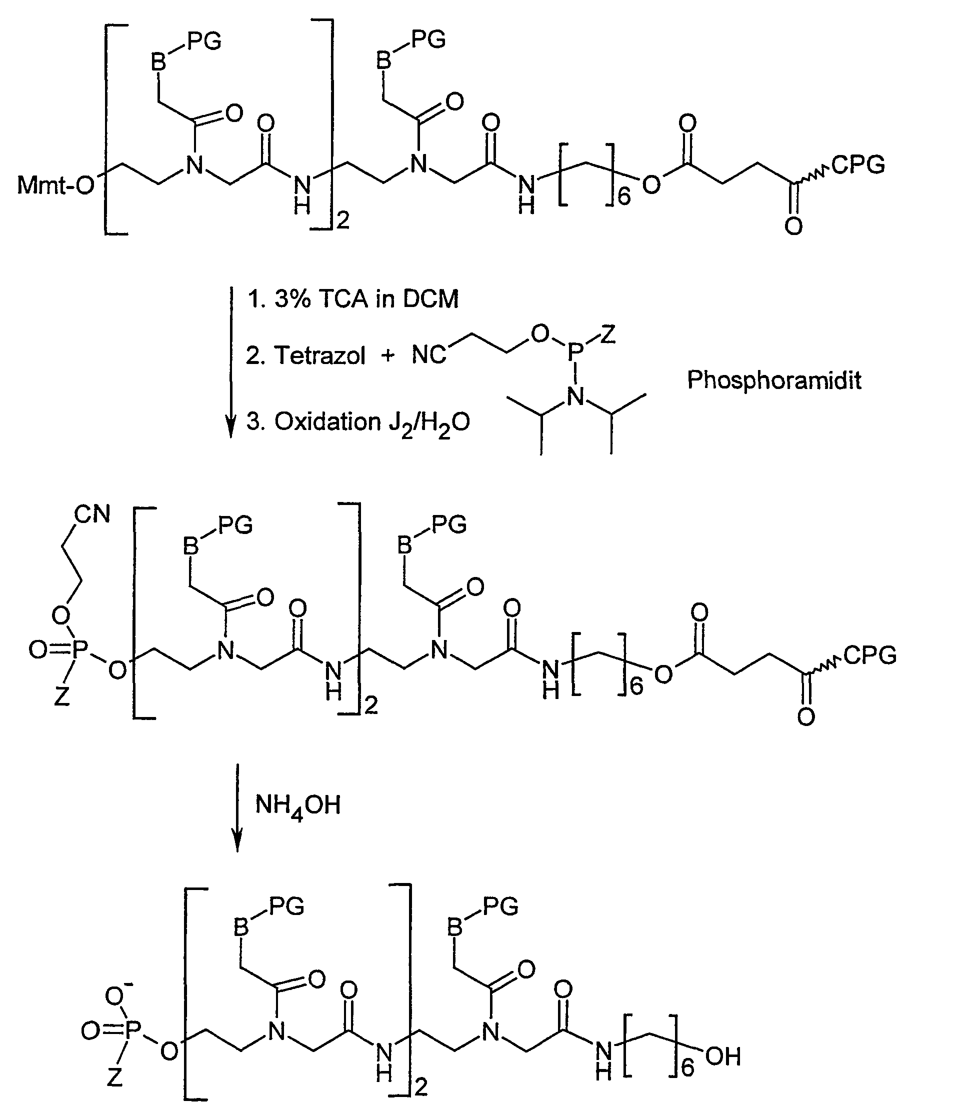

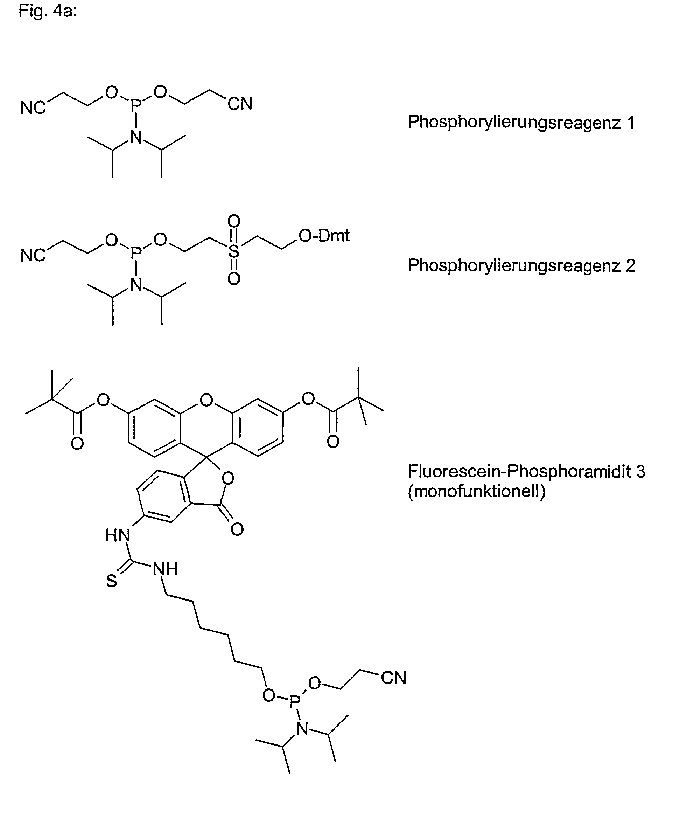

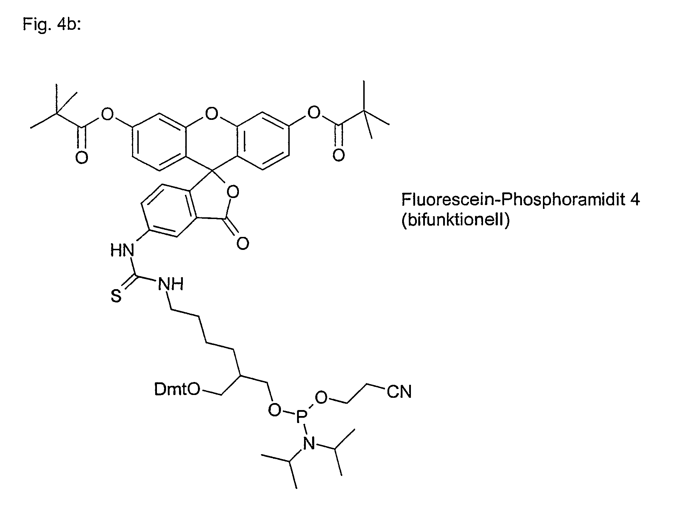

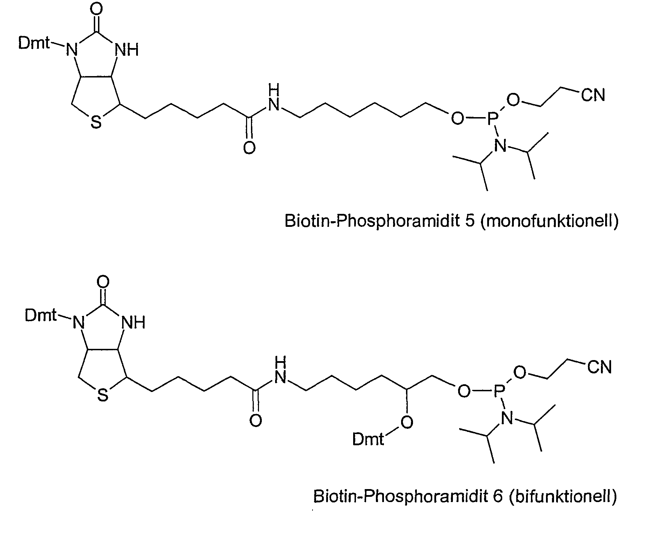

So wurde beispielsweise PNA-7 in Beispiel 8 (U = NH) durch Umsetzung des N-Terminus (am zuletzt gekuppelten Baustein mit B = Adenin) mit dem Biotin-Phosphoramidit 5 (Figur 4b) erhalten. Alternativ kann im letzten Zyklus ein Dmt-geschützter Hydroxyalkyl- (für U = O) oder Dmt-geschützter Mercaptoalkyl-Baustein (für U = S) der Formel V A gekoppelt werden. Nach Abspaltung der Dmt-Gruppe kann die freie Hydroxy- oder Mercaptofunktion beispielsweise mit dem Phosphorylierungsreagenz 1 (Figur 4a) zur Reaktion gebracht werden. Nach Oxidation, Abspaltung vom Träger und Entfernung der Schutzgruppen erhält man das Phosphat (V = W = X = Y = O) bzw. Thiophosphat (V = S, W = X = Y = O) der Formel I. Soll die aminoterminale Einheit keine Nucleobase beinhalten (B gleich Wasserstoff), so wird der Baustein gemäss Formel V B in der Kondensationsreaktion des letzten Zyklus eingesetzt, wobei die Fmoc-Schutzgruppe am Ende der Synthese mit Ammoniak abgespalten wird. Der Aminoterminus der PNA kann durch Kondensation von Bausteinen der Formel V C verlängert werden, bevor die eigentliche Phosphorylierungsreaktion durchgeführt wird.

Die Phosphoryl-Reste können mit Hilfe von in der Nucleotid-Chemie üblicherweise verwendeten Reagenzien eingeführt werden, beispielsweise durch die Phosphoramidit-Methode, die H-Phosphonat-Methode oder die Phosphotriester-Methode (E. Sonveaux (1986) Bioorganic Chemistry 14, 274; S. L. Beaucage und R. P. lyer (1993) Tetrahedron 49, 1925; E. Uhlmann und A. Peyman (1990) Chem. Rev. 90, 543). Es sind zahlreiche Phosphorylierungsreagenzien zugänglich (Glen Research Corporation, Sterling, VA 20164, U.S.A.), die zur Herstellung der Verbindungen der Formel I herangezogen werden können. Eine Auswahl der Reagenzien ist in den Figuren 4a bis 4d gezeigt, wobei die Erfindung aber nicht auf diese speziellen Derivate beschränkt ist. Bei der oben beschriebenen Phosphorylierungsreaktion kann Z auch die Bedeutung von X haben, wobei reaktive Funktionen intermediär durch geeignete Schutzgruppen geschützt sind.

Wie vielfältig die aminoterminale Modifikation ist, kann anhand der Synthese von PNA-6, PNA-12, PNA-13, und PNA-14 verdeutlicht werden. Zunächst wird die PNA t(oeg)-at tcc gtc at-hex-CPG (PNA-Basen sind durch kleine Buchstaben für die entsprechenden Nucleobasen abgekürzt; oeg steht für Hydroxyethylglycin) in vollständig geschützter Form am CPG-Träger (controlled pore glass) aufgebaut, das als letzten Baustein ein Hydroxyethylglycin-(oeg)-acetylthymin trägt. Die Hydroxygruppe kann nun individuell mit den Phosphoramiditen 1, 5, 7 bzw. 3 umgesetzt werden. Nach Oxidation, Abspaltung der Schutzgruppen und Spaltung des Produkts vom CPG-Träger erhält man entsprechend PNA-6, PNA-12, PNA-13 bzw. PNA-14. Als feste Träger werden ausserdem ®TentaGel (Fa. Rapp Polymers GmbH, Tübingen) und Aminomethylpolystyren benutzt.

Zur Einführung der Phosphoryl-Funktion kommen im Prinzip alle in der Nucleotidchemie bekannten Reagenzien in Betracht, insbesondere aber die folgenden Reagenzien der Formel VI A, Formel VI B, Formel VI C und Formel VI D 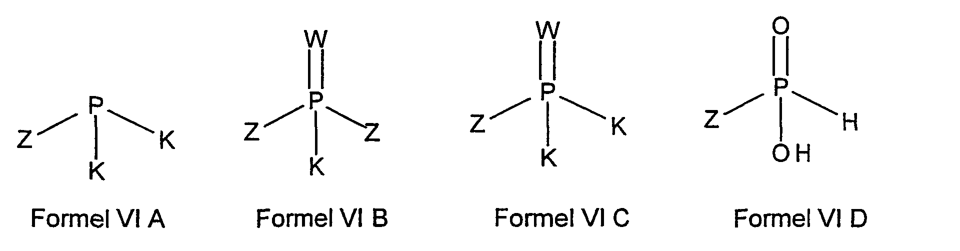 wobei K gleich Halogen, bevorzugt Cl, Triazolyl, Imidazolyl, oder Dialkylamino ist und Z die oben genannte Bedeutung oder die Bedeutung von X hat, wobei reaktive Gruppen entsprechend geschützt sind. Beispielsweise sind die Hydroxygruppen des Fluorescein-Phosphoramidits 3 (Figur 4a) durch Veresterung mit Pivalinsäure geschützt.

Die Verbindungen der Formel VI sind nur als Beispiele für solche Reagenzien zu sehen, die gegebenenfalls unter Zugabe weiterer Hilfsreagenzien wie Basen, Säuren oder Kondensationsreagenzien reagieren. Besonders bevorzugt sind die Reagenzien der Formel VI A, die nach der Phosphoramidit-Methode reagieren (Beaucage und lyer, 1993). Diese werden als Phosphor-(III)-Verbindung zur Reaktion gebracht und anschließend oxidiert. Wird die Oxidation beispielsweise mit Jod/Wasser/Pyridin oder tert-Butylhydroperoxid durchgeführt, erhält man die Phosphorylderivate (W = O). Erfolgt die Oxidation dagegen mit elementarem Schwefel oder Beaucage-Reagenz, so erhält man die entsprechende Thiophosphoryl-Verbindung (W = S).

Unter den Reagenzien (Figuren 4a bis 4d) befinden sich auch "bifunktionelle Reagenzien", die aufgrund einer zweiten Funktion, welche zunächst geschützt ist, mehrfach zur Reaktion gebracht werden können. Beispiele für solche bifunktionellen Reagenzien sind die Phosphoramidite 4, 6, 8 bis 13. Dabei kann es sich um die multiple Konjugation eines Reagenzes oder aber um die sukzessive Reaktion mit unterschiedlichen Reagenzien handeln. So kann beispielsweise das Fluorescein-Phosphoramidit 3 nur einmal zur Reaktion gebracht werden. Dagegen besitzt das Fluorescein-Phosphoramidit 4 eine durch eine Dmt-Gruppe geschützte Hydroxyfunktion, die nach Abspaltung der Dmt-Gruppe nochmals mit einem Phosphorylierungsreagenz zur Reaktion gebracht werden kann. Auf diese Weise können ein und dieselbe Gruppe oder aber unterschiedliche Gruppen mehrfach eingeführt werden. PNA-16 ist ein typisches Beispiel für eine Mehrfachkonjugation. Nach Aufbau der PNA-Kette wurde im letzten Zyklus ein Hydroxyethylglycin-C-Baustein gekoppelt, der sukzessive zweimal mit dem Spacer-18 Phosphoramidit 9, dem Aminomodifier-5 Phosphoramidit 12 und schliesslich mit Lexitropsin der Formel VII unter Aktivierung mit Peptid-Kupplungsreagenzien, wie HBTU, umgesetzt wurde. Die Kopplung eines Aminomodifiers gestattet die anschliessende Einführung eines weiteren Restes, der in Form eines aktivierten Carbonsäurederivats einführbar ist. Hierbei können beispielsweise auch Isothiocyanate und N-Hydroxysuccinimidester eingesetzt werden.

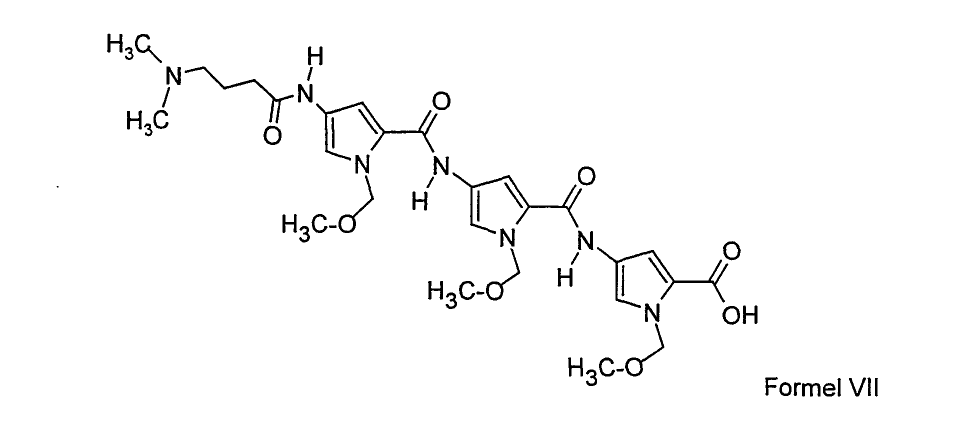

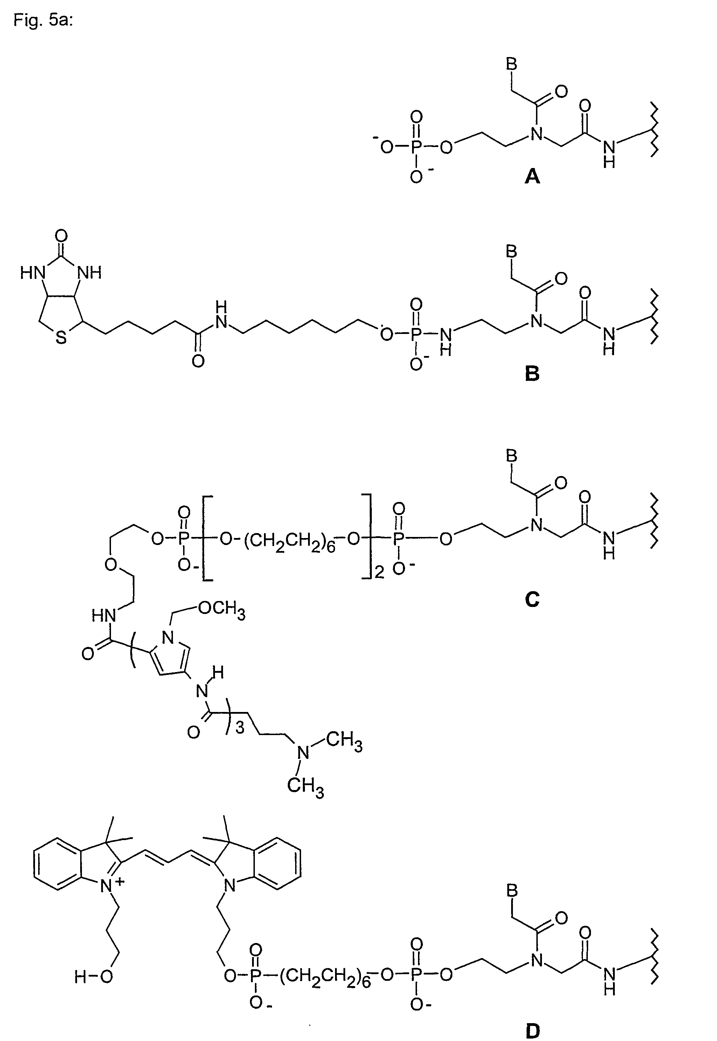

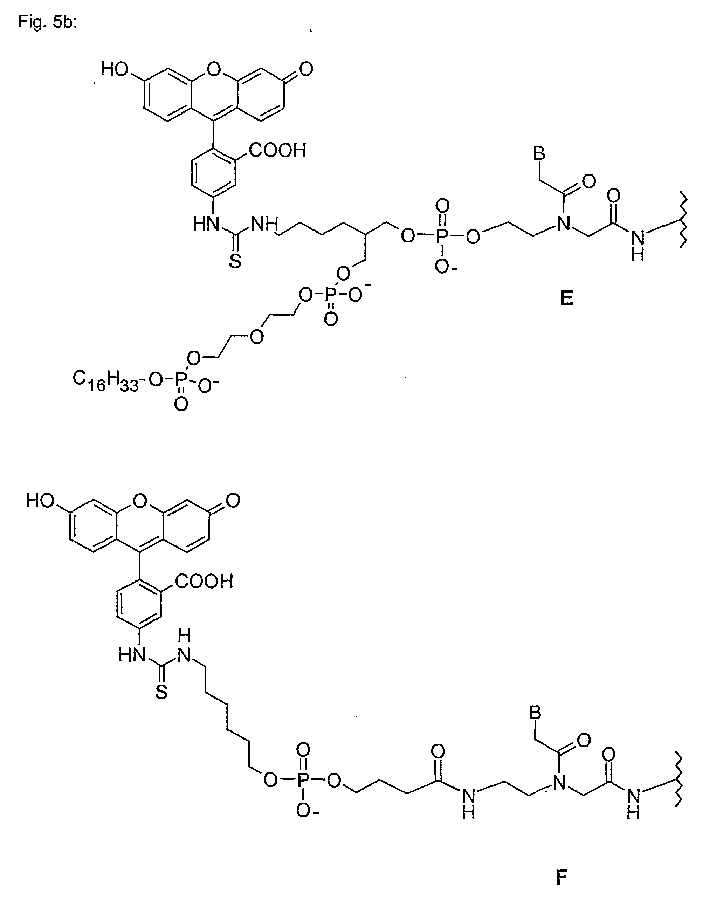

In Figur 5a und 5b sind einige Beispiele von Verbindungstypen für die N-terminale Modifikation der Verbindungen der Formel I gezeigt. Verbindungstyp A erhält man durch Reaktion der endständigen Hydroxygruppe der PNA mit dem Phosphorylierungsreagenz 1. Verbindungstyp B erhält man durch Reaktion der endständigen Aminogruppe der PNA mit dem Biotin-Phosphoramidit 5. Verbindungstyp C erhält man durch sukzessive Umsetzung der PNA mit einer endständigen Hydroxygruppe mit dem Spacer-18 Phosphoramidit 9, Aminomodifier-5 Phosphoramidit 12 und Lexitropsin. Verbindungstyp D erhält man durch sukzessive Umsetzung der PNA mit einer endständigen Hydroxygruppe mit dem Spacer-9 Phosphoramidit 8 und dem Cyanin-3 Phosphoramidit 10. Verbindungstyp E erhält man durch sukzessive Umsetzung der PNA mit einer endständigen Hydroxygruppe mit dem bifunktionellen Fluorescein-Phosphoramidit 4, dem Spacer-9 Phosphoramidit 8, und dem C16-Phosphorylierungsreagenz 7. Verbindungstyp F erhält man durch Reaktion der endständigen Aminogruppe der PNA mit 4-Hydroxybuttersäure, deren Hydroxyfunktion intermediär durch eine Dmt-Gruppe geschützt ist, und anschliessender Reaktion mit dem Fluorescein-Phosphoramidit 3. Die zusätzlich durchzuführenden Schritte, wie Oxidiation und Schutzgruppenabspaltung, sind in den Beispielen beschrieben.

### Beispiele:

Die Herstellung folgender Verbindungen ist exemplarisch beschrieben:
PNA-1 bis PNA-6: 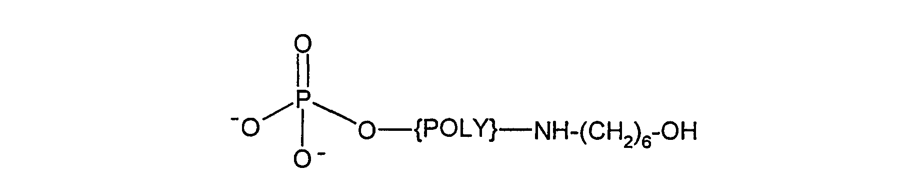
PNA-7: 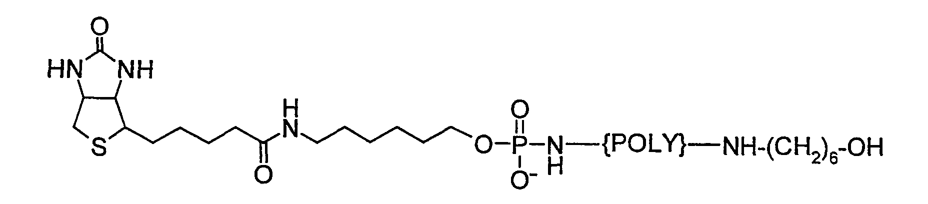
PNA-8 bis PNA-12: 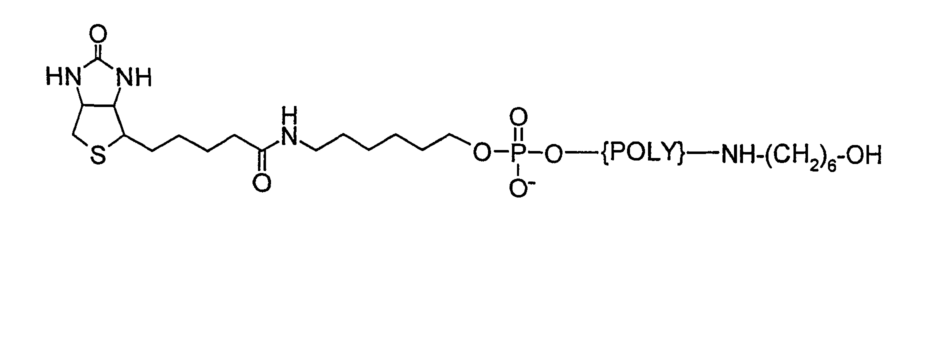
PNA-13: 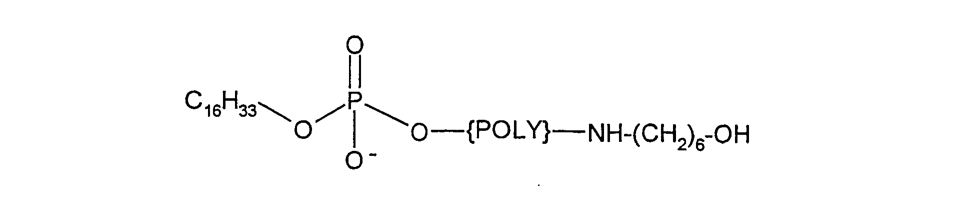
PNA-14: 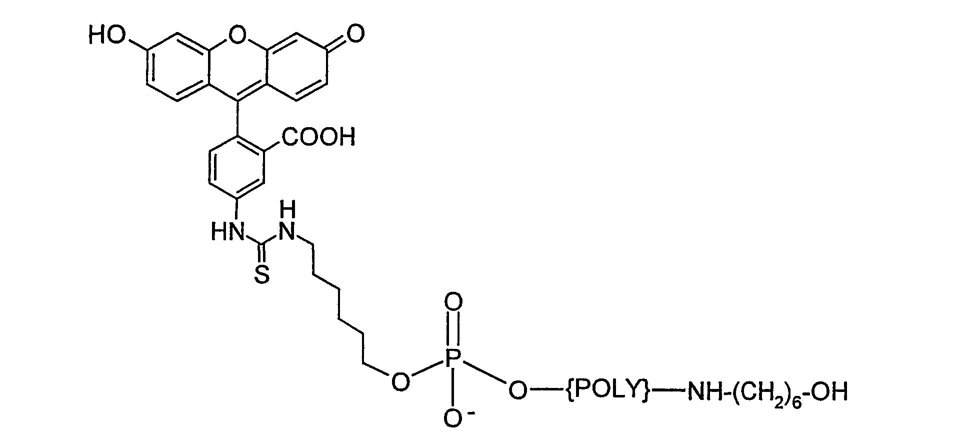
PNA-15: 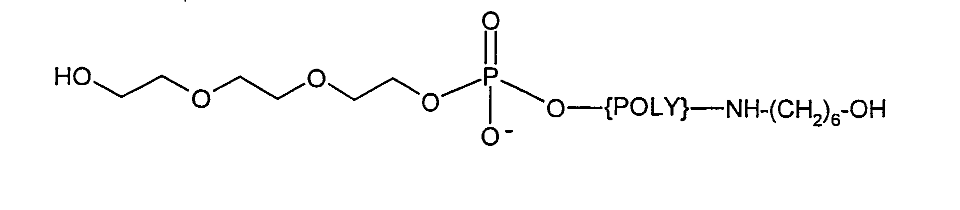
PNA-16: 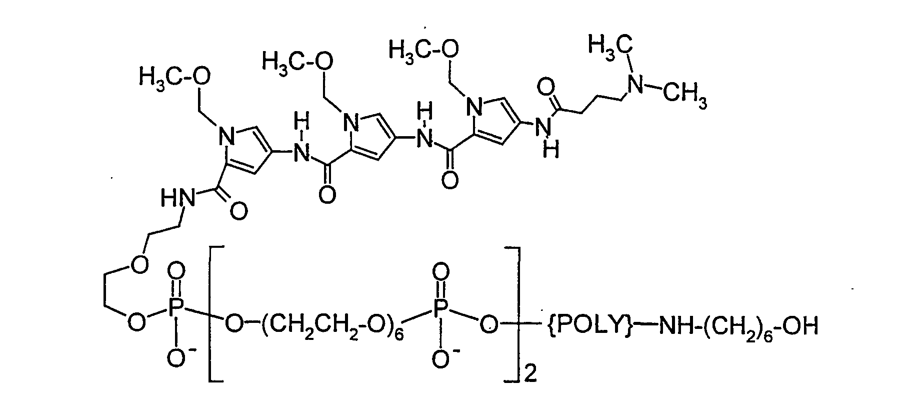
PNA-17: 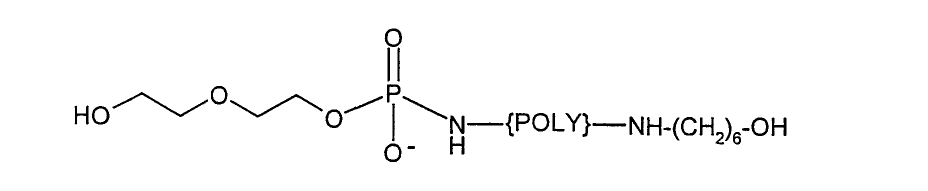
PNA-18: 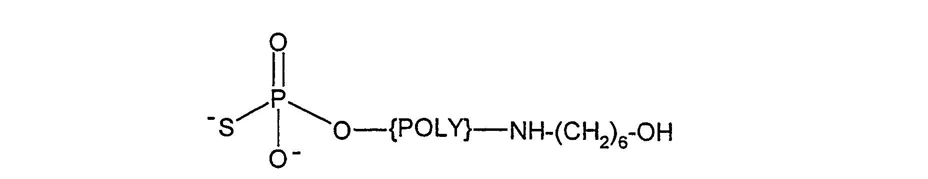 wobei die Basenabfolge wird jeweils beschrieben durch die folgenden Sequenzen, und wobei {POLY} durch Formel II beschrieben wird, {BLOCK} jeweils durch Formel IIIA beschrieben wird, und wobei ferner A und E gleich CH₂ sind und D gleich (CH₂)₂ ist, und z' sich jeweils aus der Sequenzlänge des Oligomers ergibt.

### Beispiel 1: Synthese der PNA-Kette

Zur Herstellung des PNA-Teils wurden die folgenden Reagenzien verwendet:
1. Phosphoramidit-Reagenz (0.1 M in Acetonitril (ACN))
2. Mmt-PNA-Monomere bzw. Dmt-oeg-PNA-Monomere (0.2 M in DMF:ACN (1:1; v:v))
3. Wasserfreies ACN (≤ 30 ppm Wasser)
4. Trichloressigsäure (3 %) in Dichloromethan (DCM)
5. Acetanhydrid, 2,6-Lutidin in THF (1:1:8; v:v:v); (Cap A)
6. N-Methylimidazol (16 %) in THF; (Cap B)
7. Jodlösung (0.05 M) in THF, Wasser, Pyridin, (7:2:1; v:v:v)
8. Waschlösung (THF, Wasser, Pyridin (7:2:1; v:v:v))
9. Tetrazol (0.3 M) in ACN
10. HBTU; 0.2 M in DMF:ACN (1:1; v:v)
11. DIPEA; 0.2 M in DMF:ACN (1:1; v:v)
12. DMF (> 99.5 %)
13. Festphasenträger: Aminopropyl-CPG (550 Å) beladen mit Mmt-Aminohex-1-yl hemisuccinat (für PNA-hexylamide).

Die Mmt/Acyl-geschützten bzw. Dmt/Acyl-geschützten oeg-Monomere wurden wie bereits beschrieben hergestellt (Breipohl et al. (1997) Tetrahedron 53, 14671-14686). Die Beladung von Aminopropyl-CPG mit dem Mmt-Aminohex-1-yl-hemisuccinat wurde ebenfalls bereits beschrieben (Will et al. (1995) Tetrahedron 51, 12069-12082). Die PNA-Synthesen wurden im allgemeinen im Massstab von 2 bis 5 µmol durchgeführt.

Folgender Zyklus wurde zur PNA-Synthese verwendet:
1. Waschschritt mit ACN
2. Entschützung der Mmt-Gruppe bzw. Dmt-Gruppe durch Behandlung mit 3% TCA in DCM; 110 sec.
3. Waschschritt mit DMF/ACN (1:1)
4. Neutralisierung mit DIPEA in DMF/ACN (1:1)
5. Kupplung des Monomer-Bausteins durch Voraktivierung (15 min) mit HBTU/DIPEA/PNA-Monomer (Verhältnis 1:1:1; Gesamtvolumen 450 µl) Beschickung der Festphase und Kupplung (45 min)
6. Waschschritt mit ACN
7. Capping mit Acetanhydrid/N-Methylimidazol
8. Waschschritt mit ACN
9. Neuer Zyklus

### Beispiel 2: Synthese von Phosphat-{a(oeg) act}-hex (PNA-1)

Der PNA-Teil wird wie in Beispiel 1 beschrieben durch Festphasensynthese hergestellt (2 µmol Synthese). Im letzten Zyklus wird ein auf Hydroxyethylglycin (oeg) basierender Baustein mit Adenin als Nucleobase gekuppelt (Formel V A; wobei TR gleich Dmt, U gleich Sauerstoff, u' gleich 2, PG gleich Anisoyl und B gleich Adenin ist). Nach Abspaltung der terminalen Dmt-Gruppe mit 3% TCA wird die freie Hydroxyfunktion mit dem Phosphorylierungsreagenz 1 (Figur 4a) unter Tetrazol-Katalyse zur Reaktion gebracht. Dabei werden das Phosphorylierungsreagenz 1 im Überschuss (ca. 25-fach) als 0.3 M Lösung in Acetonitril/Tetrahydrofuran (1:1; v:v) und das Tetrazol (ca. 50-fach; 0.5 M in Acetonitril) eingesetzt. Nach erfolgter Kondensation wird mit einer Jodlösung (0.05 M in Tetrahydrofuran/Wasser, Pyridin (7:2:1; v:v:v)) oxidiert. Dann wird das PNA durch Behandlung mit konz. Ammoniak bei 50°C über Nacht vom Träger gespalten und gleichzeitig die Schutzgruppen entfernt. Man erhält 83 OD (260 nm). Davon werden 40 OD durch präparative Polyacrylamid (PAA)-Gelektrophorese gereinigt. Die gewünschte Produktbande wird mit 0.2M Triethylammoniumbikarbonat-Puffer eluiert und über eine Bond-Elut C18-Säule (1 g) entsalzt. Man erhält 13.5 OD. Das Produkt wurde durch Negativionen-Massenspektrometrie analysiert, welche die berechnete Masse bestätigte (ber. 1266.2; gef. 1265.9).

### Beispiel 3: Synthese von Phosphat-{a(oeg) ca tca tgg tcg}-hex (PNA-2)

Die Herstellung erfolgt analog wie in Beispiel 2 beschrieben in einer 2 µmol Synthese. Nach der Spaltung mit Ammoniak erhält man 122 OD Rohprodukt, welches über ein 15% PAA-Gel durch Elektrophorese gereinigt wird. Man erhält 27 OD. Das Produkt wurde durch Negativionen-Massenspektrometrie analysiert, welche die berechnete Masse bestätigte (ber. 3450.3; gef. 3450.4).

### Beispiel 4: Synthese von Phosphat-{c(oeg) ca cga tga tgt}-hex (PNA-3)

Die Herstellung erfolgt analog wie in Beispiel 2 beschrieben in einer 2 µmol Synthese, wobei im letzten Zyklus ein auf Hydroxyethylglycin basierender Baustein mit Cytosin als Nucleobase gekuppelt wird. Nach der Spaltung mit Ammoniak erhält man 124 OD Rohprodukt, welches über ein 15% PAA-Gel durch Elektrophorese gereinigt wird. Man erhält 19 OD. Das Produkt wurde durch Negativionen-Massenspektrometrie analysiert, welche die berechnete Masse bestätigte (ber. 3450.3; gef. 3450.1).

### Beispiel 5: Synthese von Phosphat-{g(oeg)-ag cca tgt ata gtg ac}-hex (PNA-4)

Die Herstellung erfolgt analog wie in Beispiel 2 beschrieben in einer 2 µmol Synthese, wobei im letzten Zyklus ein auf Hydroxyethylglycin basierender Baustein mit Guanin als Nucleobase gekuppelt wird. Nach der Spaltung mit Ammoniak erhält man 120 OD Rohprodukt. 60 OD des Rohprodukts wurden über ein 15% PAA-Gel durch Elektrophorese gereinigt wird. Man erhält 10 OD. Das Produkt wurde durch Negativionen-Massenspektrometrie analysiert, welche die berechnete Masse bestätigte (ber. 4848.6; gef. 4849.9).

### Beispiel 6: Synthese von Phosphat-{t(oeg) cg gtt tga gat ctg g}-hex (PNA-5)

Die Herstellung erfolgt analog wie in Beispiel 2 beschrieben in einer 2 µmol Synthese, wobei im letzten Zyklus ein auf Hydroxyethylglycin basierender Baustein mit Thymin als Nucleobase gekuppelt wird. Nach der Spaltung mit Ammoniak erhält man 250 OD Rohprodukt. 60 OD des Rohprodukts wurden über ein 15% PAA-Gel durch Elektrophorese gereinigt wird. Man erhält 22.9 OD. Das Produkt wurde durch Negativionen-Massenspektrometrie analysiert, welche die berechnete Masse bestätigte (ber. 4595.4; gef. 4596.3).

### Beispiel 7: Synthese von Phosphat-{t(oeg)-at tcc gtc at}-hex (PNA-6)

Die Herstellung erfolgt analog wie in Beispiel 2 beschrieben in einer 0.5 µmol Synthese, wobei im letzten Zyklus ein auf Hydroxyethylglycin basierender Baustein mit Thymin als Nucleobase gekuppelt wird. Nach der Spaltung mit Ammoniak erhält man 63 OD Rohprodukt. 30 OD des Rohprodukts wurden über ein 15% PAA-Gel durch Elektrophorese gereinigt wird. Man erhält 4.2 OD. Das Produkt wurde durch Negativionen-Massenspektrometrie analysiert, welche die berechnete Masse bestätigte (ber. 3124.5; gef. 3124.8).

### Beispiel 8: Synthese von Biotin-p-{act gat gta gtc}-hex (PNA-7)

Der PNA-Teil wird wie in Beispiel 1 beschrieben durch Festphasensynthese hergestellt (2 µmol Synthese). Im letzten Zyklus wird ein normaler PNA-Baustein mit Adenin als Nucleobase gekuppelt (Formel V A; wobei TR gleich Mmt, U gleich NH, u' gleich 2, PG gleich Anisoyl und B gleich Adenin ist). Nach Abspaltung der terminalen Mmt-Gruppe mit 3% TCA wird die freie Aminofunktion mit dem Biotin-Phosphoramidit 5 (Figur 4b) unter Tetrazol-Katalyse zur Reaktion gebracht. Dabei werden das Phosphorylierungsreagenz im Überschuss (ca. 10-fach) als 0.12 M Lösung in Acetonitril und das Tetrazol (ca. 50-fach; 0.5 M in Acetonitril) eingesetzt. Nach erfolgter Kondensation wird mit einer Jodlösung (0.05 M in Tetrahydrofuran/Wasser, Pyridin (7:2:1; v:v:v)) oxidiert. Nach der Spaltung mit Ammoniak erhält man 288 OD Rohprodukt. Das Rohprodukt wurde über ein 15% PAA-Gel durch Elektrophorese gereinigt. Man erhält 17.5 OD. Das Produkt wurde durch Negativionen-Massenspektrometrie analysiert, welche die berechnete Masse bestätigte (ber. 3789.6; gef. 3789.8).

### Beispiel 9: Synthese von Biotin-p-{g(oeg)-ct gat gta gtc}-hex (PNA-8)

Der PNA-Teil wird wie in Beispiel 1 beschrieben durch Festphasensynthese hergestellt (1 µmol Synthese). Im letzten Zyklus wird ein auf Hydroxyethylglycin basierender Baustein mit Guanin als Nucleobase gekuppelt (Formel V A; wobei TR gleich Dmt, U gleich Sauerstoff, u' gleich 2, PG gleich Anisoyl und B gleich Guanin ist). Nach Abspaltung der terminalen Dmt-Gruppe mit 3% TCA wird die freie Hydroxyfunktion mit dem Biotin-Phosphoramidit 5 (Figur 4b) unter Tetrazol-Katalyse zur Reaktion gebracht. Dabei werden das Phosphorylierunsreagenz im Überschuss (ca. 10-fach) als 0.12 M Lösung in Acetonitril und das Tetrazol (ca.-50-fach; 0.5 M in Acetonitril) eingesetzt. Nach erfolgter Kondensation wird mit einer Jodlösung (0.05 M in Tetrahydrofuran/Wasser, Pyridin (7:2:1; v:v:v)) oxidiert. Nach der Spaltung mit Ammoniak erhält man 63 OD Rohprodukt. Das Rohprodukt wurde über ein 15% PAA-Gel durch Elektrophorese gereinigt. Man erhält 11.2 OD. Das Produkt wurde durch Negativionen-Massenspektrometrie analysiert, welche die berechnete Masse bestätigte (ber. 3806.8; gef. 3807.2).

### Beispiel 10: Synthese von Biotin-p-{g(oeg) gt atg gga tat}-hex (PNA-9)

Die Herstellung erfolgte analog wie in Beispiel 9 beschrieben in einer 2 µmol Synthese, wobei im letzten Zyklus ein auf Hydroxyethylglycin basierender Baustein mit Guanin als Nucleobase gekuppelt wird. Der Biotin-Rest wurde mit dem Biotin-Phosphoramidit 5 (Figur 4b) eingeführt. Nach der Spaltung mit Ammoniak erhält man 274 OD Rohprodukt. Das Rohprodukt wurde über ein 15% PAA-Gel durch Elektrophorese gereinigt wird. Man erhält 25.8 OD. Das Produkt wurde durch Negativionen-Massenspektrometrie analysiert, welche die berechnete Masse bestätigte (ber. 3870.8; gef. 3869.7).

### Beispiel 11: Synthese von Biotin-p-{t(oeg) ga agg aag agg}-hex (PNA-10)

Die Herstellung erfolgte analog wie in Beispiel 9 beschrieben in einer 2 µmol Synthese, wobei im letzten Zyklus ein auf Hydroxyethylglycin basierender Baustein mit Thymin als Nucleobase gekuppelt wird. Der Biotin-Rest wurde mit dem Biotin-Phosphoramidit 5 (Figur 4b) eingeführt. Nach der Spaltung mit Ammoniak erhält man 190 OD Rohprodukt. Das Rohprodukt wurde über ein 12% PAA-Gel durch Elektrophorese gereinigt wird. Man erhält 29 OD mit dem erwarteten Molekulargewicht (ber. 3913.9; gef. 3913.7).

### Beispiel 12: Synthese von Biotin-p-{g(oeg)-gt atg gga tat}-hex (PNA-11)

Die Herstellung erfolgte analog wie in Beispiel 9 beschrieben in einer 2 µmol Synthese, wobei im letzten Zyklus ein auf Hydroxyethylglycin basierender Baustein mit Guanin als Nucleobase gekuppelt wird. Der Biotin-Rest wurde mit dem Biotin-Phosphoramidit 5 (Figur 4b) eingeführt. Nach der Spaltung mit Ammoniak erhält man 162 OD Rohprodukt. Das Rohprodukt wurde über ein 12% PAA-Gel durch Elektrophorese gereinigt wird. Man erhält 21 OD. Das Produkt wurde durch Negativionen-Massenspektrometrie analysiert, welche die berechnete Masse bestätigte (ber. 3870.8; gef. 3870.8).

### Beispiel 13: Synthese von Biotin-p-{t(oeg) at tcc gtc at}-hex (PNA-12)

Die Herstellung erfolgte analog wie in Beispiel 9 beschrieben in einer 0.5 µmol Synthese, wobei im letzten Zyklus ein auf Hydroxyethylglycin basierender Baustein mit Thymin als Nucleobase gekuppelt wird. Der Biotin-Rest wurde mit dem Biotin-Phosphoramidit 5 (Figur 4b) eingeführt. Nach der Spaltung mit Ammoniak erhält man 67 OD Rohprodukt. Das Rohprodukt wurde über ein 12% PAA-Gel durch Elektrophorese gereinigt wird. Man erhält 8.5 OD. Das Produkt wurde durch Negativionen-Massenspektrometrie analysiert, welche die berechnete Masse bestätigte (ber. 3449.5; gef. 3449.9).

### Beispiel 14: Synthese von Hexadecyl-O-p-{t(oeg) at tcc gtc at}-hex (PNA-13)

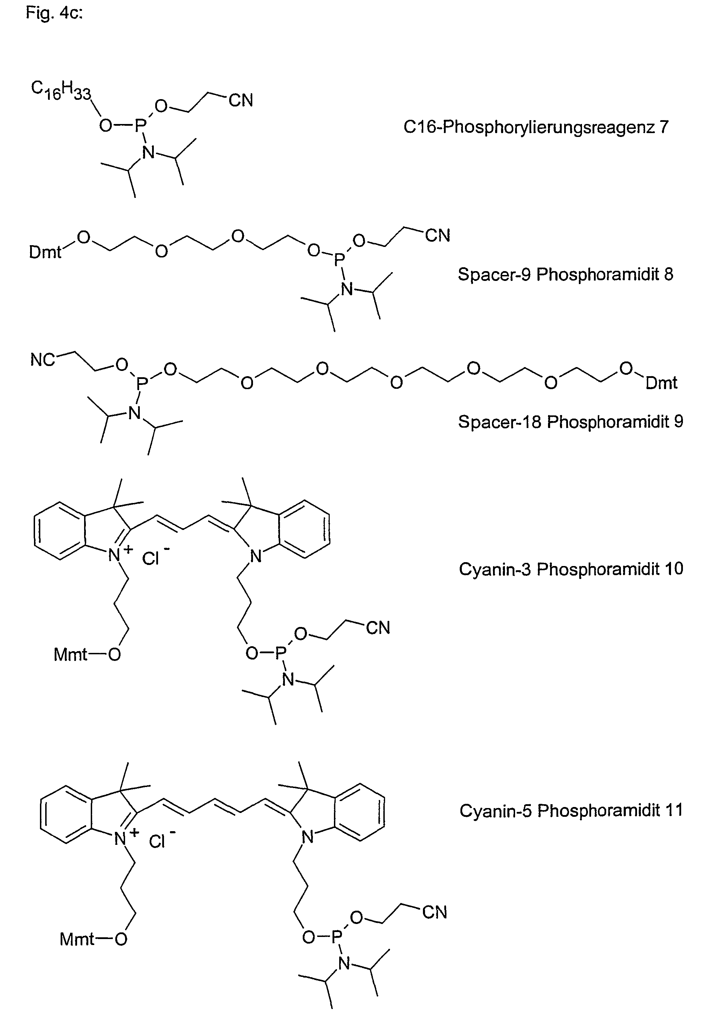

Die Herstellung erfolgte analog wie in Beispiel 9 beschrieben in einer 0.5 µmol Synthese, wobei im letzten Zyklus ein auf Hydroxyethylglycin basierender Baustein mit Thymin als Nucleobase gekuppelt wird. Der Hexadecyl-Rest wurde mit dem C16-Phosphorylierungsreagenz 7 (Figur 4c) eingeführt. Nach der Spaltung mit Ammoniak erhält man 58 OD Rohprodukt. Das Rohprodukt (30 OD) wurde über ein 12% PAA-Gel durch Elektrophorese gereinigt wird. Man erhält 2 OD mit dem erwarteten Molekulargewicht (ber. 3349.4; gef. 3349.7).

### Beispiel 15: Synthese von Fluorescein-p-{t(oeg) at tcc gtc at}-hex (PNA-14)

Die Herstellung erfolgte analog wie in Beispiel 9 beschrieben in einer 0.5 µmol Synthese, wobei im letzten Zyklus ein auf Hydroxyethylglycin basierender Baustein mit Thymin als Nucleobase gekuppelt wird. Der Fluorescein-Rest wurde mit dem Fluorescein-Phosphoramidit 3 (Figur 4a) eingeführt. Nach der Spaltung mit Ammoniak erhält man 62 OD Rohprodukt. Das Rohprodukt (30 OD) wurde über ein 12% PAA-Gel durch Elektrophorese gereinigt wird. Man erhält 4.2 OD. Das Produkt wurde durch Negativionen-Massenspektrometrie analysiert, welche die berechnete Masse bestätigte (ber. 3581.5; gef. 3582.4).

### Beispiel 16: Synthese von HO-spacer9-p-{g(oeg)-tt agg gtt ag}-hex (PNA-15)

Die Herstellung erfolgte analog wie in Beispiel 9 beschrieben in einer 1 µmol Synthese, wobei im letzten Zyklus ein auf Hydroxyethylglycin basierender Baustein mit Thymin als Nucleobase gekuppelt wird. Der Spacer-9 wurde mit dem entsprechenden Phosphoramidit 8 (Figur 4c) eingeführt. Nach der Spaltung mit Ammoniak erhält man 52 OD Rohprodukt. Das Rohprodukt (30 OD) wurde über ein 15% PAA-Gel durch Elektrophorese gereinigt wird. Man erhält 1.8 OD. Das Produkt wurde durch Negativionen-Massenspektrometrie analysiert, welche die berechnete Masse bestätigte (ber. 3402.3; gef. 3401.8).

### Beispiel 17: Synthese von Lexitropsin-aminolinkC5-p-spacerC18-p-spacerC18-p-{c(oeg)-c cct tcc}-hex (PNA-16; wobei c gleich ein pseudo-iso-Cytosin PNA-Baustein ist)

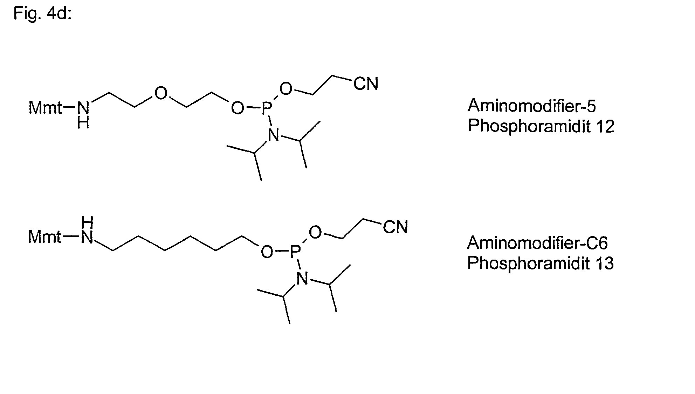

Die Herstellung erfolgte analog wie in Beispiel 9 beschrieben in einer 1 µmol Synthese, wobei im letzten Zyklus ein auf Hydroxyethylglycin basierender Baustein mit Cytosin als Nucleobase gekuppelt wird. Dann werden nacheinander zweimal das Spacer-18 Phosphoramidit 9 und einmal das Aminomodifier-5 Phosphoramidit 12 (Figur 4d) kondensiert. Nach den Kupplungsreaktionen wurde die Dmt- bzw. Mmt-Gruppe jeweils durch Behandlung mit 3% Trichloressigsäure abgespalten. Zur Kupplung des Lexitropsins gibt man 300 µl des entsprechenden Aktivesters zu und läßt 3 Stunden reagieren (hergestellt aus 0.1 M Lexitropsincarbonsäure, 0.1 M TBTU, und 0.4M Diisopropylethylamin; 1:1:1 =v:v:v; 30 min. Voraktivierung). Danach wird mit DMF gewaschen. Nach der Spaltung mit Ammoniak erhält man 43 OD Rohprodukt. Das Rohprodukt (35 OD) wurde über ein 15% PAA-Gel durch Elektrophorese gereinigt wird. Man erhält 5.3 OD. Das Produkt wurde durch Negativionen-Massenspektrometrie analysiert, welche die berechnete Masse bestätigte (ber. 3583.5; gef. 3583.1).

### Beispiel 18: Bestimmung der Schmelztemperaturen

Die Bestimmung der Schmelztemperaturen erfolgte mit Hilfe eines HP 8452A Diodenarray-Spektrophotometers, eines HP 89090A Peltier-Elements und der HP Temperature Control Software Rev. B5.1 (Fa. Hewlett Packard). Es wird in 0.5°C/min Schritten in 140 mM KCI, 10 mM Natriumdihydrogenphosphat, 0.1mM EDTA (pH 7.4) als Puffer gemessen. Die Oligomerkonzentration beträgt 0.5 bis 1 OD₂₆₀ pro ml.

Überraschenderweise zeigten die phosphorylmodifzierten PNA-6, PNA-12 bis PNA-14 Derivate eine höhere Bindung gegenüber komplementärer DNA und RNA als die ungeladene PNA (Referenzsubstanz).

| PNA-Derivat | | Tₘ (DNA) | Tₘ (RNA) |
|---|---|---|---|
| Referenz | Ac-HN-tat tcc gtc at-hex | 41.9°C | 56.6°C |
| PNA-6 | p-{t(oeg)-at tcc gtc at}-hex | 44.0°C | 57.5°C |
| PNA-13 | Hexadecyl-O-p-{t(oeg) at tcc gtc at}-hex | 46.7°C | 58.9°C |
| PNA-14 | Fluorescein-p-{t(oeg) at tcc gtc at}-hex | 42.5°C | 56.7°C |
| PNA-12 | Biotin-p-{t(oeg) at tcc gtc at}-hex | 43.6°C | 57.9°C |

### Beispiel 19: Bestimmung der Zellaufnahme nach Fluoreszenz-Markierung

Man läßt die COS-Zellen bis zur Konfluenz in Dulbecco's MEM (DMEM), das mit 10% FCS supplementiert wurde, in 5 cm Petrischalen heranwachsen. Die Zellen werden zweimal mit serumfreien DMEM gewaschen. Mit Hilfe einer sterilen Nadel wird eine Fläche von ca. 1 cm² der Mitte der Petrischale eingekratzt. In diese Fläche wird die zu untersuchende PNA-Lösung (10 µM) aufgebracht. Es wird bei 37°C unter CO₂-Atmosphäre inkubiert. Nach 2, 4 und 16 Stunden werden die Zellen durch Fluoreszenzmikroskopie untersucht. Dazu werden die Zellen viermal mit serumfreien DMEM gewaschen, mit einem Glasträger abgedeckt und unter dem Fluoreszenzmikroskop bzw. durch Phasenkontrast bewertet.

Zur Untersuchung der Zellaufnahme wurden PNA-6 und PNA-13 am C-terminus mit Fluorescein markiert und mikroskopisch untersucht.
p-{t(oeg)-at tcc gtc at}-Fluorescein
Hexadecyl-O-p-{t(oeg) at tcc gtc at}-Fluorescein

Dabei zeigte sich, dass das Hexadecyl-PNA-Derivat (PNA-13) effizienter in die Zellen aufgenommen wurde.

### Beispiel 20: Hemmung der Zellproliferation durch PNA-13

Die Sequenz von PNA-13 ist gegen den Translationsstart der Ha-ras mRNA gerichtet. Die REH-Zellen (human pre-B leukemia cells, DSM ACC 22) oder A549-Tumorzellen wurden in OptiMEM (Gibco BRL) mit 10 % fötalem Kälberserum (FCS, GIBCO-BRL) bei 37°C unter 5% CO₂ kultiviert. Die Zelldichte für den Assay war ca. 1 x 106 / ml). Die PNA-13 (10 µM) wurde mit den Zellen in 24-well Platten inkubiert. Nach 96 Inkubation bei 37°C unter 5% CO₂ wurde die Zelldichte bestimmt. Mittelwerte der Zelldichte wurden aus 3 individuellen Löchern einer PNA Konzentration ermittelt. Es zeigte sich, dass PNA-13 die Proliferation der REH Zellen hemmt. Nach > 4 Tagen Inkubationszeit ist die Hemmung durch PNA-13 stärker als durch ein entsprechendes Phosphorothioat-Oligonucleotid.

### Beispiel 21: Synthese von HO-spacer9-p-{gtt agg gtt ag}-hex (PNA-17)

Die Herstellung erfolgte analog wie in den Beispielen 9 und 16 beschrieben in einer 0.67 µmol Synthese, wobei im letzten Zyklus der PNA-Synthese ein normaler 2-Amino-ethylglycin Baustein mit Thymin als Nucleobase gekuppelt wird. Anschließend wurde der Spacer-9 mit dem entsprechenden Phosphoramidit 8 (Figur 4c) eingeführt (3 x 5 min Reaktionszeit). Nach der Spaltung mit Ammoniak erhält man 108 OD Rohprodukt. Das Rohprodukt wurde über ein 15% PAA-Gel durch Elektrophorese gereinigt wird. Man erhält 5.7 OD. Das Produkt wurde durch Negativionen-Massenspektrometrie analysiert, welche die berechnete Masse bestätigte (ber. 3401.3; gef. 3399.8).

### Beispiel 22: Synthese von Thiophosphat-{t(oeg)-at tcc gtc at}-hex (PNA-18)

Die Herstellung erfolgt analog wie in den Beispielen 2 und 7 beschrieben in einer 1 µmol Synthese, wobei im letzten Zyklus ein auf Hydroxyethylglycin basierender Baustein mit Thymin als Nucleobase gekuppelt wird. Nach Abspaltung der terminalen Dmt-Gruppe mit 3% TCA wird die freie Hydroxyfunktion mit dem Phosphorylierungsreagenz 1 (Figur 4a) unter Tetrazol-Katalyse zur Reaktion gebracht. Nach erfolgter Kondensation wird zur Einführung der Thio-Funktion mit dem Beaucage-Reagenz (0.075 M 3H-1,2-Benzodithiole-3-one 1,1-dioxide in Acetonitril) oxidiert. Nach der Spaltung mit Ammoniak erhält man 66.5 OD Rohprodukt. 61 OD des Rohprodukts wurden über ein 15% PAA-Gel durch Elektrophorese gereinigt wird. Man erhält 12.4 OD. Das Produkt wurde durch Negativionen-Massenspektrometrie analysiert, welche die berechnete Masse bestätigte (ber. 3141; gef. 3140).

### Abkürzungsverzeichnis:

- ACN: Acetonitril
- BOC: tert-Butyloxycarbonyl
- C, c: pseudo-iso-Cytosin
- COS: CV1 Origin SV 40
- CPG: controlled pore glass
- DCM: Dichlormethan
- DIPEA: Diisopropylethylamin
- DMEM: Dulbecco's MEM
- DMF: Dimethylformamid
- Dmt: Dimethoxytrityl
- DNA: Desoxyribonukleinsäure
- DNP: Dinitroaryl
- FITC: Fluoresceinisothiocyanat
- Fmoc: Fluorenylmethoxycarbonyl
- HATU: O-(7-Azabenzotriazol-1-yl)-1,1,3,3-tetramethyluroniumhexafluorphosphat
- HBTU: O-(Benzotriazol-1-yl)-1,1,3,3-tetramethyluroniumhexafluorphosphat

-NH-(CH₂)₆-OH
- hex MEM: Modified Eagle's minimal essential medium
- Mmt: Monomethoxytrityl
- OD: Optische Dichte
- oeg: N-(2-Hydroxyethyl)glycin
- PAA: Polyacrylamid
- PG: Schutzgruppe
- PNA: Polyamidnukleinsäure
- RNA: Ribonukleinsäure
- TBTU: O-(Benzotriazol-1-yl)-1,1,3,3-tetramethyluronium-tetrafluoroborat
- TCA: Trichloressigsäure
- THF: Tetrahydrofuran
- TR: säurelabile Schutzgruppe

Figuren 1a, 1 b, 2b und 3b zeigen Beispiele für endständige Reste Z.

Figuren 2a und 3a zeigen Beispiele für verbrückende Reste X.

Figuren 4a, 4b, 4c und 4d zeigen Beispiele für Phosphorylierungsreagenzien.

Figuren 5a und 5b zeigen Beispiele für die einfache (A, B) und multiple (C bis F) N-terminale Derivatisierung von PNA.

## Patentansprüche

1. PNA-Derivat der Formel I 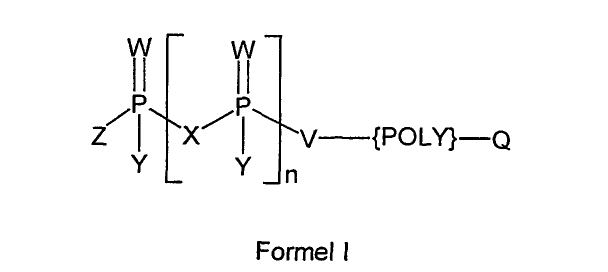 wobei
V gleich Sauerstoff, Schwefel, NR_{1,} eine Gruppe U-(CR₃R₄)_{u'}-CH₂-C(O)-NH oder eine Gruppe U-(CH₂CH₂O)_{u'}-C(O)-NH ist,
U unabhängig voneinander gleich Sauerstoff, Schwefel oder NH ist,
u' unabhängig voneinander gleich 1 bis 10 ist, vorzugsweise 1 bis 4, besonders bevorzugt 1,
W unabhängig voneinander gleich Sauerstoff, Schwefel oder NR₁ ist,
Y unabhängig voneinander gleich Hydroxy, Mercapto, Oxyanion, Thioat oder NR₁R₂ ist,
R₁ und R₂ unabhängig voneinander einen Rest bestehend aus Wasserstoff oder C₁-C₆-Alkyl bedeuten, vorzugsweise Wasserstoff,
R₃ und R₄ unabhängig voneinander einen Rest bestehend aus Wasserstoff oder C₁-C₆-Alkyl oder den Rest einer Aminosäure-Seitenkette bedeuten, vorzugsweise Wasserstoff,
X unabhängig voneinander gleich eine Gruppe U-(C₂-C₂₂-Alkandiyl)-U oder eine Gruppe U-(CH₂CH₂-O)_{u'} ist,
oder X gleich eine bifunktionelle Markergruppe ist, bevorzugt aus der Gruppe der Fluorescein-, Rhodamin-, TAMRA-, Biotin-, Pyren-, Dinitrophenyl-, Acridin-, Cyaninfarbstoff-, Dabcyl-, Digoxigenin- oder Edans-Derivate, besonders bevorzugt ein Biotin-Derivat,
oder X gleich eine bifunktionelle Gruppe zur Quervernetzung mit komplementären Nukleinsäuren ist, bevorzugt ein Psoralen-Derivat,
oder X gleich eine bifunktionelle Gruppe ist, die die intrazelluläre Aufnahme begünstigt, bevorzugt aus der Gruppe der Cholesteryl-, Adamantyl- und Vitamin-E-Derivate,
oder X gleich eine bifunktionelle Gruppe ist, die die Bindungsaffinität des PNA-Derivates an eine Target-Nukleinsäure steigert, bevorzugt Acridin- und Lexitropsin-Derivate,
Z gleich Hydroxy, Mercapto, Oxyanion, Thioat oder NR₁R₂, C₁-C₂₂-Alkyl, C₁-C₈-Arylalkyl, C₁-C₂₂-Alkyl-U, C₁-C₈-Arylalkyl-U, Hydroxy-C₁-C₁₈-U, Aminoalkyl-U, Arylalkyl-U, Mercaptoalkyl-U ist, oder eine Gruppe der Formel R₉(CH₂CH₂-O)ₘ ist, wobei R₉ gleich Hydroxy, Amino oder C₁-C₂₂-Alkoxy und m gleich 1 bis 100 ist, vorzugsweise 2 bis 10,
oder Z gleich eine monofunktionelle oder bifunktionelle Markergruppe ist, bevorzugt aus der Gruppe der Fluorescein-, Rhodamin-, TAMRA-, Biotin-, Pyren-, Dinitrophenyl-, Acridin-, Cyaninfarbstoff-, Dabcyl-, Digoxigenin- oder Edans-Derivate, besonders bevorzugt ein Biotin-Derivat,
oder Z gleich eine monofunktionelle oder bifunktionelle quervernetzenden Gruppe ist, bevorzugt ein Psoralen-Derivat,
oder Z gleich eine monofunktionelle oder bifunktionelle Gruppe ist, die die intrazelluläre Aufnahme begünstigt, bevorzugt aus der Gruppe der Cholesteryl-, Adamantyl- und Vitamin-E-Derivate,
oder Z gleich eine monofunktionelle oder bifunktionelle Gruppe ist, die die Bindungsaffinität des PNA-Derivates an eine target-Nukleinsäure steigert, bevorzugt ein Lexitropsin-Derivat,
n gleich 0 bis 10 ist, vorzugsweise 0 bis 3,
Q gleich Hydroxy, Amino, NHR₇, NR₇R₈, Aminosäure-Derivat oder ein Peptid-Rest ist,
R₇ und R₈ unabhängig voneinander C₁-C₁₈-Alkyl oder Hydroxy-C₁-C₁₈-Alkyl sind,
und wobei {POLY} beschrieben wird durch die Formel II 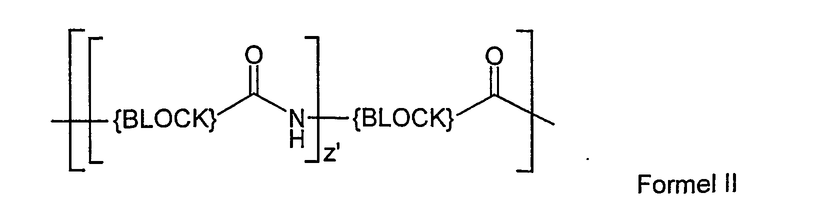 wobei {BLOCK} unabhängig voneinander entweder beschrieben wird durch Formel IIIA, 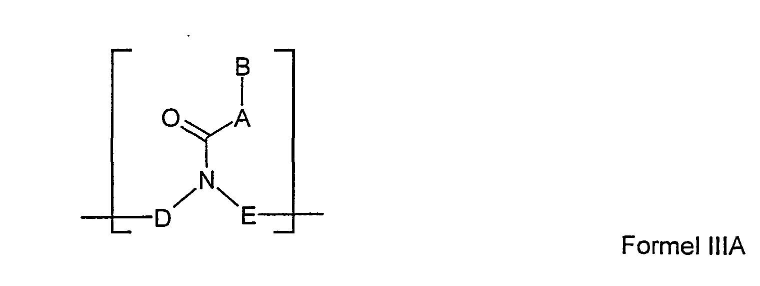 oder Formel IIIB, 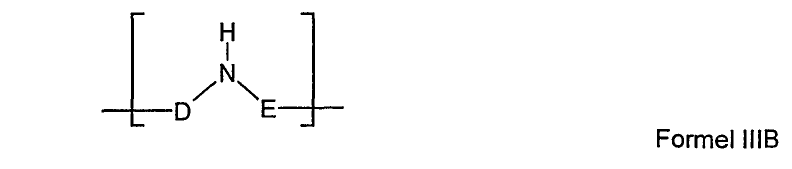 oder Formel IV A bis IV G, 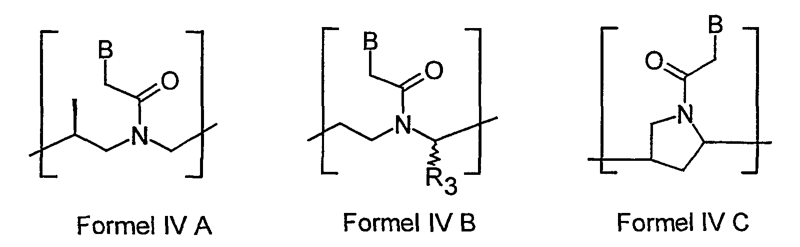 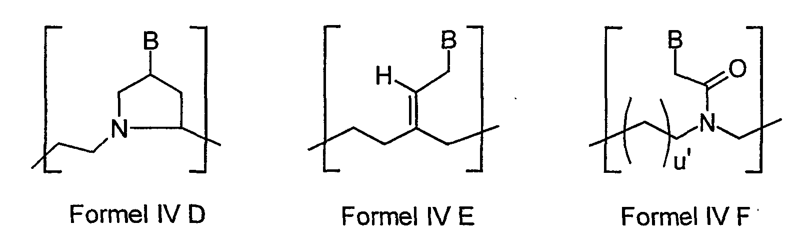 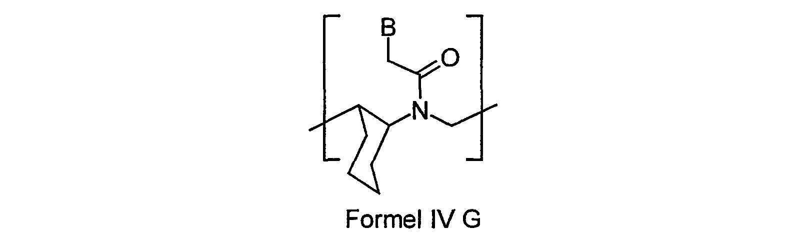 wobei jeder Baustein {BLOCK} verschieden sein kann, und wobei ferner gilt, dass
z' gleich 0 bis 100 ist, vorzugsweise 1-20, besonders bevorzugt 4-15
A unabhängig voneinander eine Gruppe (CR₁R₂)ₛ ist, wobei s gleich 1 bis 3 ist, vorzugsweise 1,
B unabhängig voneinander entweder ein aromatischer Rest, der auch heteroaromatischen Charakter besitzen kann, oder Wasserstoff, oder Hydroxy oder C₁-C₁₈-Alkyl ist,
oder eine in der Nucleotidchemie übliche natürlich vorkommende oder eine nicht natürlich vorkommende Nucleobase oder deren Prodrugform ist,
wobei mindestens ein Rest B eine Nucleobase ist,
D unabhängig voneinander eine Gruppe (CR₃R₄)ₜ ist, wobei t gleich 2 bis 10 ist, vorzugsweise 2 bis 4, besonders bevorzugt 2, wobei zwei benachbarte Reste R₃ und R₄ auch einen C₅-C₈-Cycloalkylring bilden können,
E unabhängig voneinander eine Gruppe (CR₅R₆)_{u'} ist, wobei zwei benachbarten Reste R₅ und R₆ auch einen C₅-C₈-Cycloalkyl-Ring oder eine Spiroverbindung bilden können.
R₅ und R₆ unabhängig voneinander einen Rest bestehend aus Wasserstoff oder C₁-C₆-Alkyl sind, vorzugsweise Wasserstoff, oder eine Aminosäure-Seitenkette sind.
sowie physiologisch verträglichen Salze des PNA-Derivates der Formel I,
mit der Maßgabe, dass mindestens ein Rest Y oder Z gleich Hydroxy, Mercapto, Oxyanion oder Thioat ist.

2. PNA-Derivat nach Anspruch 1, wobei mindestens ein Rest Y oder Z in Formel I in einem pH-Bereich von 4,5 bis 14, vorzugsweise 6,5 bis 12, besonders bevorzugt 6,5 bis 9 gleich Oxyanion oder Thioat ist.

3. PNA-Derivat nach einem der Ansprüche 1 bis 2, wobei D gleich (CH₂)₂ ist.

4. PNA-Derivat nach einem der Ansprüche 1 bis 3, wobei A und E gleich CH₂ sind.

5. PNA-Derivat nach einem der Ansprüche 1 bis 4, wobei Q gleich ein Hydroxyaminoalkyl-Rest ist, bevorzugt ein Hydroxyaminohexyl-Rest, oder gleich einer Carriersequenz ist, bevorzugt Transportan, Insulin-like Growth Factor, Nuclear Localisation Signale, oder ein Affinitäts-Tag ist, bevorzugt eine (His)₆ Kette.

6. PNA-Derivat nach einem der Ansprüche 1 bis 5, wobei B gleich Adenin, Cytosin, 5-Methylcytosin, Guanin, Thymin und Uracil, oder gleich Purin, 2,6-Diaminopurin, N⁴N⁴-Ethanocytosin, N⁶N⁶-Ethano-2,6-diaminopurin, 5-(C₃-C₆)-Alkinyl-uracil, 5-(C₃-C₆)-Alkinyl-cytosin, 5-(1-Propargylamino)-uracil, 5-(1-Propargylamino)-cytosin, Phenoxazin, 9-Aminoethoxyphenoxazin, 5-Fluor-uracil oder Pseudoisocytosin, 5-(Hydroxymethyl)uracil, 5-Aminouracil, Pseudouracil, Dihydrouracil, 5-(C₁-C₆)-Alkyl-uracil, 5-(C₁-C₆)-Alkyl-cytosin, 5-(C₂-C₆)-Alkenyl-cytosin, 5-Fluorcytosin, 5-Chloruracil, 5-Chlorcytosin, 5-Bromuracil, 5-Bromcytosin, 7-Deazaadenin, 7-Deazaguanin, 8-Azapurin, oder ein 7-Deaza-7-substituiertes Purin ist.

7. PNA-Derivat nach einem der Ansprüche 1 bis 6, wobei W gleich Sauerstoff, und Y gleich Hydroxy oder Oxyanion sind.

8. PNA-Derivat nach einem der Ansprüche 1 bis 7, wobei X gleich eine Gruppe U-(C₂-C₂₂-Alkandiyl)-U, bevorzugt O-(C₂-C₂₂-Alkandiyl)-O, besonders bevorzugt O-(CH₂)₂₋₆O ist, oder gleich eine Gruppe U-(CH₂CH₂-O)_{u'}, bevorzugt O-(CH₂CH₂-O)_{u'}, wobei u' bevorzugt 1 bis 6 ist.

9. PNA-Derivat nach einem der Ansprüche 1 bis 8, wobei Z gleich Phosphat oder ein C₁- bis C₂₂-Rest ist, oder ein C₁-C₂₂-U-Rest, bevorzugt ein C₁-C₂₂-Alkoxy-Rest, besonders bevorzugt C₁₆-Alkoxy, oder Hydroxy-C₁-C₁₈-U, bevorzugt Hydroxy-C₁-C₁₈-O, besonders bevorzugt HO-(CH₂)₃₋₁₂O, oder ein Aminoalkyl-U-Rest, bevorzugt ein Aminoalkoxy-Rest, besonders bevorzugt 6-Aminohexoxy oder 5-Aminopentoxy, oder eine Gruppe der Formel R₉-(CH₂CH₂-O)ₘ ist, wobei R₉ bevorzugt OH oder NH₂ ist und m gleich 1 bis 6 ist, besonders bevorzugt HO(CH₂CH₂-O)₂, HO(CH₂CH₂-O)₆ und H₂N-(CH₂CH₂-O)₂, oder ein Mercaptoalkyl-U-Rest, bevorzugt ein Mercaptoalkoxy-Rest, besonders bevorzugt 6-Mercaptohexyloxy.

10. PNA-Derivat nach Anspruch 1 bis 7, wobei X und Z unabhängig voneinander ausgewählt werden aus der Gruppe Biotin-, Fluorescein- oder Lexitropsin-Derivat ist.

11. PNA-Derivat nach einem der Ansprüche 1 bis 7, wobei X und Z unabhängig voneinander ausgewählt werden aus der Gruppe Rhodamin, TAMRA oder Cyanin-Farbstoff.

12. PNA-Derivat nach einem der Ansprüche 1 bis 7, wobei X und Z unabhängig voneinander ausgewählt werden aus der Gruppe Dabcyl, Psoralen, Acridin, DNP oder Cholesterol.

13. PNA-Derivat nach einem der Ansprüche 1 bis 13, wobei die Basensequenz gegen Teile von Tumorsupressor-Genen, Onkogenen oder Telomerasen oder deren Transkriptions-Produkte gerichtet ist.

14. PNA-Derivat nach Anspruch 13, wobei die Basensequenz des PNA-Teils gegen den Translationsstart von HA-ras mRNA gerichtet ist.

15. PNA-Derivat nach einem der Ansprüche 1 bis 14 zur Verwendung als Arzneimittel.

16. Verwendung eines PNA-Derivates nach einem der Ansprüche 1 bis 14 zur Herstellung eines Arzneimittels für die Tumortherapie.

17. PNA-Derivat nach einem der Ansprüche 1 bis 14 zur Verwendung als Diagnostikum.

18. Verwendung eines PNA-Derivats nach einem der Ansprüche 1 bis 14 zum Nachweis von Mikroorganismen und/oder Viren in vitro.

19. Verwendung eines PNA-Derivats nach einem der Ansprüche 1 bis 14 zum Nachweis und/oder Quantifizierung von Nukleinsäuren in vitro.

20. Verwendung eines PNA-Derivats nach einem der Ansprüche 1 bis 14 als Nachweisreagenz für die in-situ- oder Fluoreszenz-in-situ-Hybridisierung in vitro.

21. Verwendung eines PNA-Derivats nach einem der Ansprüche 1 bis 14 als Antisense-, Anti-Gen-, Decoy-, oder Chimeraplast-Agenz in vitro.

22. Nachweisreagenz enthaltend ein PNA-Derivat nach einem der Ansprüche 1 bis 14.

23. PNA-Chip, enthaltend ein PNA-Derivat nach einem der Ansprüche 1 bis 14.

24. Blosensor enthaltend ein PNA-Derivat nach einem der Ansprüche 1 bis 14.

25. Arzneimittel, enthaltend ein PNA-Derivat nach einem der Ansprüche 1 bis 14 und gegebenenfalls weitere pharmakologisch verträgliche Zusatz- und/oder Trägerstoffe.

26. Antisense-Agenz, enthaltend ein PNA-Derivat nach einem der Ansprüche 1 bis 14.

27. Verfahren zur Herstellung eines PNA-Derivates nach einem der Ansprüche 1 bis 14, wobei
a) das PNA-Rückgrat vom C-Terminus ausgehend mittels aktivierter Amidnukleinsäuren aufgebaut wird, und
b) am N-Terminus mit einem Phosphorylierungsreagenz umgesetzt wird.

28. Verfahren nach Anspruch 27, wobei die PNA unter Verwendung der Schutzgruppen t-Butyloxycarbonyl (BOC), 9-Fluorenylmethoxycarbonyl (Fmoc) oder Monomethoxytrityl (Mmt) hergestellt wird.

29. Verfahren nach Anspruch 28, wobei die PNA unter Verwendung von festen Trägern hergestellt wird.

30. Verfahren nach Anspruch 29, wobei als fester Träger CPG, Tentagel oder Aminomethylpolystyren verwendet wird.

31. Verfahren zur Herstellung eines Arzneimittels, wobei
a) ein PNA-Derivat gemäß einem der Ansprüche 27 bis 30 hergestellt wird, und
b) gegebenfalls mit weiteren pharmakologisch verträglichen Zusatz- und/oder Trägerstoffen versetzt wird.

32. Verfahren zur Herstellung eines PNA-Chips, wobei gemäß einem der Ansprüche 27 bis 30 entweder zuerst ein PNA-Derivat hergestellt und dann auf einem festen Träger fixiert wird, oder das PNA-Derivat direkt auf dem Träger hergestellt wird.

33. Verfahren zur Herstellung eines PNA-Derivats der Formel I gemäß Anspruch 27 bis 30, ferner **dadurch gekennzeichnet, dass** die PNA unter Ausnutzung des sauren Charakters des Phosporrestes mittels Chromatographie oder Elektrophorese aufgereinigt wird. '

34. Verfahren nach Anspruch 33, wobei ein PNA-Derivat durch Chromatographie mittels einer basischen stationären Phase und einem sauren oder salzhaltigen Eluent gereinigt werden.

35. Verfahren nach Anspruch 34, wobei die stationäre Phase ein Anionenaustauscher oder eine Mixed-Mode-Phase ist.

## Claims

1. A PNA derivative of the formula I 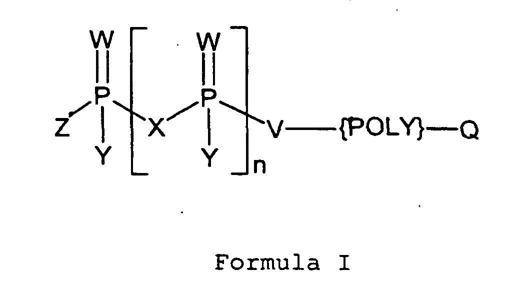 where
V is oxygen, sulfur, NR₁, a group U-(CR₃R₄)_{u'}-CH₂-C(O)-NH or a group U-(CH₂CH₂O)_{u'}-C(O)-NH,
U is, independently of each other, oxygen, sulfur or NH,
u' is, independently of each other, from 1 to 10, preferably from 1 to 4, particularly preferably 1,
W is, independently of each other, oxygen, sulfur or NR₁,
Y is, independently of each other, hydroxyl, mercapto, oxyanion, thioate or NR₁R₂,
R₁ and R₂ are, independently of each other, a radical consisting of hydrogen or C₁-C₆-alkyl, preferably hydrogen,
R₃ and R₄ are, independently of each other, a radical consisting of hydrogen or C₁-C₆-alkyl, or the radical of an amino acid side chain, preferably hydrogen,
X is, independently of each other, a group U-(C₂-C₂₂-alkanediyl)-U or a group U-(CH₂CH₂-O)_{u'}, or X is a bifunctional labeling group, preferably selected from the group consisting of fluorescein, rhodamine, TAMRA, biotin, pyrene, dinitrophenyl, acridine, cyanine dye, Dabcyl, digoxigenin or Edans derivatives, particularly preferably a biotin derivative,
or X is a bifunctional group for crosslinking with complementary nucleic acids, preferably a psoralen derivative,
or X is a bifunctional group which promotes intracellular uptake, preferably selected from the group consisting of cholesteryl, adamantyl and vitamin E derivatives,
or X is a bifunctional group which increases the binding affinity of the PNA derivative for a target nucleic acid, preferably acridine and lexitropsin derivatives,
Z is hydroxyl, mercapto, oxyanion, thioate or NR₁R₂, C₁-C₂₂-alkyl, C₁-C₈-arylalkyl, C₁-C₂₂-alkyl-U, C₁-C₈-arylalkyl-U, hydroxy-C₁-C₁₈-U, aminoalkyl-U, arylalkyl-U or mercaptoalkyl-U,
or a group of the formula R₉(CH₂CH₂-O)ₘ , where R₉ is hydroxyl, amino or C₁-C₂₂-alkoxy, and m is from 1 to 100, preferably from 2 to 10,
or Z is a monofunctional or bifunctional labeling group, preferably selected from the group consisting of fluorescein, rhodamine, TAMRA, biotin, pyrene, dinitrophenyl, acridine, cyanine dye, Dabcyl, digoxigenin or Edans derivatives, particularly preferably a biotin derivative,
or Z is a monofunctional or bifunctional crosslinking group, preferably a psoralen derivative,
or Z is a monofunctional or bifunctional group which promotes intracellular uptake, preferably selected from the group consisting of cholesteryl, adamantyl and vitamin E derivatives,
or Z is a monofunctional or bifunctional group which promotes the binding affinity of the PNA derivative for a target nucleic acid, preferably a lexitropsin derivative,
n is from 0 to 10, preferably from 0 to 3,
Q is hydroxyl, amino, NHR₇, NR₇R₈, an amino acid derivative or a peptide radical,
R₇ and R₈ are, independently of each other, C₁-C₁₈-alkyl or hydroxy-C₁-C₁₈-alkyl,
and where {POLY} is described by the formula II 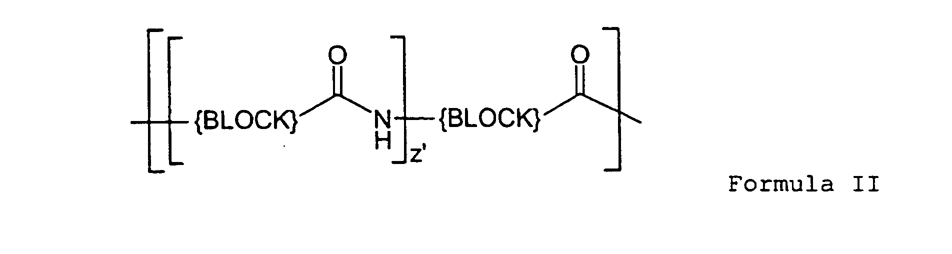 where {BLOCK} is, independently of each other, either described by formula IIIA, 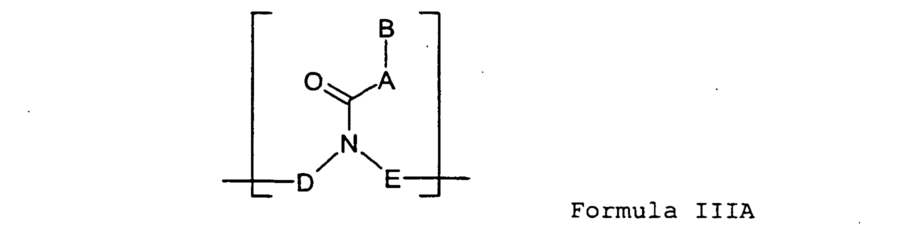 or formula IIIB, 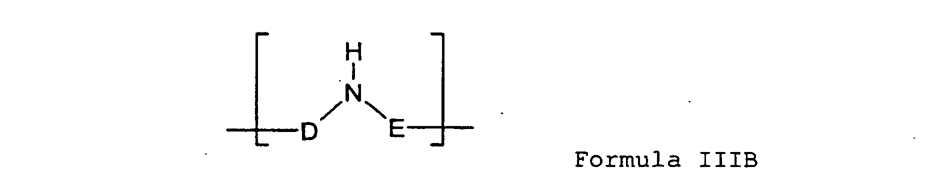 or formulae IV A to IV G, 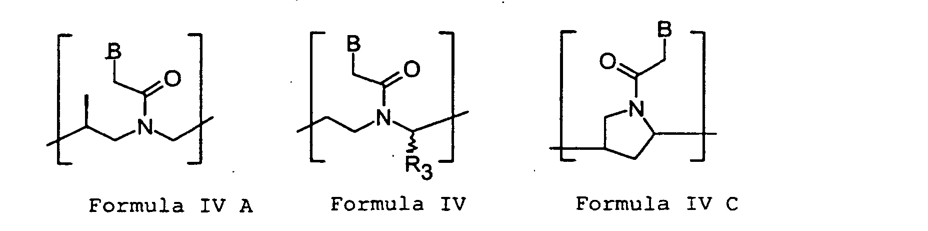 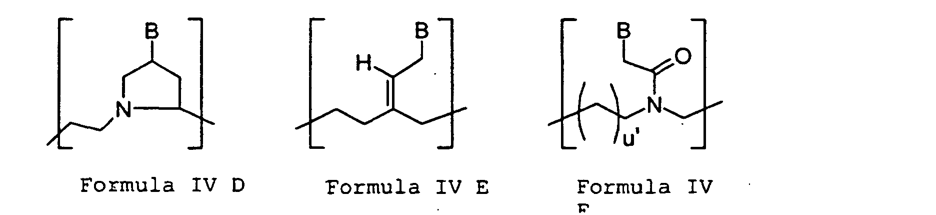 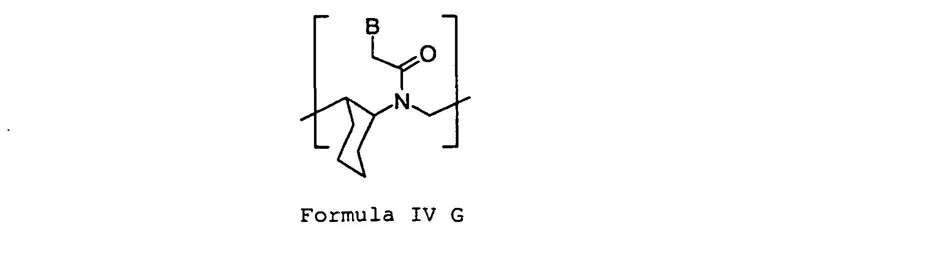 where each building block {BLOCK} can be different,
and where it furthermore applies that
z' is from 0 to 100, preferably 1-20, particularly preferably 4-15,
A is, independently of each other, a group (CR₁R₂)ₛ, where s is from 1 to 3, preferably 1,
B is, independently of each other, either an aromatic radical, which can also possess heteroaromatic character, or hydrogen, or hydroxyl or C₁-C₁₈-alkyl,
or a nucleobase which occurs naturally, and is customary in nucleotide chemistry, or which does not occur naturally, or its prodrug form,
where at least one B radical is a nucleobase,
D is, independently of each other, a group (CR₃R₄)ₜ,
where t is from 2 to 10, preferably from 2 to 4, particularly preferably 2,
where two adjacent R₃ and R₄ radicals can also form a C₅-C₈-cycloalkyl ring,
E is, independently of each other, a group (CR₅R₆)_{u'},
where two adjacent R₅ and R₆ radicals can also form a C₅-C₈-cycloalkyl ring or a spiro compound,
R₅ and R₆ are, independently of each other, a radical consisting of hydrogen or C₁-C₆-alkyl, preferably hydrogen, or an amino acid side chain,
and also physiologically tolerated salts of the PNA derivative of the formula I,
with the proviso that at least one Y or Z radical is hydroxyl, mercapto, oxyanion or thioate.

2. A PNA derivative as claimed in claim 1, wherein at least one Y or Z radical in formula I is oxyanion or thioate in a pH range from 4.5 to 14, preferably from 6.5 to 12, particularly preferably from 6.5 to 9.

3. A PNA derivative as claimed in either claim 1 or 2, wherein D is (CH₂)₂.

4. A PNA derivative as claimed in one of claims 1 to 3, wherein A and E are CH₂.

5. A PNA derivative as claimed in one of claims 1 to 4, wherein Q is a hydroxyaminoalkyl radical, preferably a hydroxyaminohexyl radical, or is a carrier sequence, preferably transportan, insulin-like growth factor, nuclear localization signals or an affinity tag, preferably a (His)₆ chain.

6. A PNA derivative as claimed in one of claims 1 to 5, wherein B is adenine, cytosine, 5-methylcytosine, guanine, thymine or uracil, or is purine, 2,6-diaminopurine, N⁴N⁴-ethanocytosine, N⁶N⁶-ethano-2,6-diaminopurine, 5-(C₃-C₆)-alkynyluracil, 5-(C₃-C₆)-alkynylcytosine, 5-(1-propargylamino)uracil, 5-(1-propargylamino)cytosine, phenoxazine, 9-aminoethoxyphenoxazine, 5-fluorouracil or pseudoisocytosine, 5-(hydroxymethyl)uracil, 5-aminouracil, pseudouracil, dihydrouracil, 5-(C₁-C₆)-alkyluracil, 5- (C₁-C₆)-alkylcytosine, 5- (C₂-C₆)-alkenylcytosine, 5-fluorocytosine, 5-chlorouracil, 5-chlorocytosine, 5-bromouracil, 5-bromocytosine, 7-deazaadenine, 7-deazaguanine, 8-azapurine or a 7-deaza-7-substituted purine.

7. A PNA derivative as claimed in one of claims 1 to 6, wherein W is oxygen and Y is hydroxyl or oxyanion.

8. A PNA derivative as claimed in one of claims 1 to 7, wherein X is a group U-(C₂-C₂₂-alkanediyl)-U, preferably O-(C₂-C₂₂-alkanediyl)-O, particularly preferably O-(CH₂)₂₋₆O, or is a group U-(CH₂CH₂-O)_{u'}, preferably O(CH₂CH₂-O)_{u'}, where u' is preferably from 1 to 6.

9. A PNA derivative as claimed in one of claims 1 to 8, wherein Z is a phosphate or a C₁- to C₂₂-radical, or a C₁-C₂₂-U radical, preferably a C₁-C₂₂-alkoxy radical, particularly preferably C₁₆-alkoxy, or hydroxy-C₁-C₁₈-U, preferably hydroxy-C₁-C₁₈-O, particularly preferably HO- (CH₂)₃₋₁₂O, or an aminoalkyl-U radical, preferably an aminoalkoxy radical, particularly preferably 6-aminohexoxy or 5-aminopentoxy, or a group of the formula R₉-(CH₂CH₂-O)ₘ, where R₉ is preferably OH or NH₂ and m is from 1 to 6, particularly preferably HO(CH₂CH₂-O)₂, HO(CH₂CH₂-O)₆ and H₂N-(CH₂CH₂-O)₂, or a mercaptoalkyl-U radical, preferably a mercaptoalkoxy radical, particularly preferably 6-mercaptohexyloxy.

10. A PNA derivative as claimed in claims 1 to 7, wherein X and Z are, independently of each other, selected from the group consisting of biotin, fluorescein and lexitropsin derivatives.

11. A PNA derivative as claimed in one of claims 1 to 7, wherein X and Z are selected, independently of each other, from the group consisting of rhodamine, TAMRA and cyanine dye.

12. A PNA derivative as claimed in one of claims 1 to 7, wherein X and Z are selected, independently of each other, from the group consisting of Dabcyl, psoralen, acridine, DNP and cholesterol.

13. A PNA derivative as claimed in one of claims 1 to 13, wherein the base sequence is directed against parts of tumor suppressor genes, oncogenes or telomerases, or their transcription products.

14. A PNA derivative as claimed in claim 13, wherein the base sequence of the PNA moiety is directed against the translation start of HA-ras mRNA.

15. A PNA derivative as claimed in one of claims 1 to 14 for use as a pharmaceutical.

16. The use of a PNA derivative as claimed in one of claims 1 to 14 for producing a pharmaceutical for tumor therapy.

17. A PNA derivative as claimed in one of claims 1 to 14 for use as a diagnostic agent.

18. The use of a PNA derivative as claimed in one of claims 1 to 14 for detecting microorganisms and/or viruses in vitro.

19. The use of a PNA derivative as claimed in one of claims 1 to 14 for detecting and/or quantifying nucleic acids in vitro.

20. The use of a PNA derivative as claimed in one of claims 1 to 14 as a detection reagent for in-situ hybridization or fluorescence in-situ hybridization in vitro.

21. The use of a PNA derivative as claimed in one of claims 1 to 14 as an antisense agent, anti-gene agent, decoy agent or chimeraplast agent in vitro.

22. A detection reagent, comprising a PNA derivative as claimed in one of claims 1 to 14.

23. A PNA chip, comprising a PNA derivative as claimed in one of claims 1 to 14.

24. A biosensor, comprising a PNA derivative as claimed in one of claims 1 to 14.

25. A pharmaceutical, comprising a PNA derivative as claimed in one of claims 1 to 14 and, where appropriate, other pharmacologically tolerated additives and/or excipients.

26. An antisense agent, comprising a PNA derivative as claimed in one of claims 1 to 14.

27. A process for preparing a PNA derivative as claimed in one of claims 1 to 14, wherein
a) the PNA backbone is synthesized, starting from the C terminus, using activated amidonucleic acids, and
b) a reaction with a phosphorylating reagent is carried out at the N terminus.

28. The process as claimed in claim 27, wherein the PNA is prepared using the t-butyloxycarbonyl (BOC), 9-fluorenylmethoxycarbonyl (Fmoc) or monomethoxytrityl (Mmt) protecting groups.

29. The process as claimed in claim 28, wherein the PNA is prepared using solid supports.

30. The process as claimed in claim 29, wherein CPG, tentagel or aminomethylpolystyrene is used as the solid support.

31. A process for producing a pharmaceutical, wherein
a) a PNA derivative as claimed in one of claims 27 to 30 is prepared, and
b) further pharmacologically tolerated additives and/or excipients are added to it, where appropriate.

32. A process for producing a PNA chip, wherein, as claimed in one of claims 27 to 30, a PNA derivative is firstly prepared and then fixed onto a solid support, or the PNA derivative is prepared directly on the support.

33. A process for preparing a PNA derivative of the formula I as claimed in claims 27 to 30, wherein, in addition, the PNA is purified by means of chromatography or electrophoresis while exploiting the acid character of the phosphorus radical.

34. The process as claimed in claim 33, wherein a PNA derivative is purified by means of chromatography using a basic stationary phase and an acidic or salt-containing eluent.

35. The process as claimed in claim 34, wherein the stationary phase is an anion exchanger or a mixed-mode phase.

## Revendications

1. Dérivé d'acide polyamidonucléique (PNA) de formule I 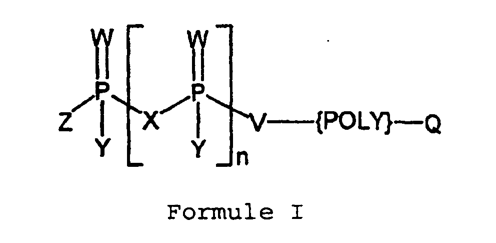 dans laquelle
V représente oxygène, soufre, NR₁, un groupe U-(CR₃R₄)_{u'} -CH₂-C(O)-NH ou un groupe U-(CH₂CH₂O)_{u'}-C(O)-NH,
U représente, indépendamment l'un de l'autre, oxygène, soufre ou NH,
u' signifie, indépendamment l'un de l'autre, 1 à 10, de préférence 1 à 4, de manière particulièrement préférée 1,
W représente, indépendamment l'un de l'autre, oxygène, soufre ou NR₁,
Y représente, indépendamment l'un de l'autre, hydroxy, mercapto, oxyanion, thioate ou NR₁R₂,
R₁ et R₂ signifient, indépendamment l'un de l'autre, un radical constitué par hydrogène ou alkyle en C₁ à C₆, de préférence hydrogène,
R₃ et R₄ signifient, indépendamment l'un de l'autre, un radical constitué par hydrogène ou alkyle en C₁ à C₆ ou le résidu d'une chaîne latérale d'acide aminé, de préférence hydrogène,
X représente, indépendamment l'un de l'autre, un groupe U-((alcane en C₂ à C₂₂)diyle)-U ou un groupe U-(CH₂CH₂-O)_{u'},
ou X représente un groupe marqueur difonctionnel, de préférence du groupe des dérivés de fluorescéine, rhodamine, TAMRA, biotine, pyrène, dinitrophényle, acridine, colorant cyanine, dabcyle, digoxygénine ou Edans, de manière particulièrement préférée un dérivé de biotine,
ou X représente un groupe difonctionnel pour la réticulation transversale avec des acides nucléiques complémentaires, de préférence un dérivé de psoralène,
ou X représente un groupe difonctionnel qui favorise l'absorption intracellulaire, de préférence du groupe des dérivés cholestéryle, adamantyle et de vitamine E,
ou X représente un groupe difonctionnel qui augmente l'affinité de liaison du dérivé de PNA pour un acide nucléique cible, de préférence des dérivés d'acridine et de lexitropsine,
Z représente hydroxy, mercapto, oxyanion, thioate ou NR₁R₂, alkyle en C₁ à C₂₂, arylalkyle en C₁ à C₈, (alkyle en C₁ à C₂₂)-U, (aralkyle en C₁ à C₈)-U, hydroxy-(C₁ à C₁₈)-U, aminoalkyl-U, arylalkyl-U, mercaptoalkyl-U, ou un groupe de formule R₉(CH₂CH₂-O)ₘ, où R₉ représente hydroxy, amino ou alcoxy en C₁ à C₂₂ et m représente 1 à 100, de préférence 2 à 10,
ou Z représente un groupe marqueur monofonctionnel ou difonctionnel, de préférence du groupe des dérivés de fluorescéine, rhodamine, TAMRA, biotine, pyrène, dinitrophényle, acridine, colorant cyanine, dabcyle, digoxygénine ou Edans, de manière particulièrement préférée un dérivé de biotine,
ou Z représente un groupe monofonctionnel ou difonctionnel à réticulation transversale, de préférence un dérivé de psoralène,
ou Z représente un groupe monofonctionnel ou difonctionnel, qui favorise l'absorption intracellulaire, de préférence du groupe des dérivés cholestéryle, adamantyle et de vitamine E,
ou Z représente un groupe monofonctionnel ou difonctionnel qui augmente l'affinité de liaison du dérivé de PNA pour un acide nucléique cible, de préférence un dérivé de lexitropsine,
n vaut 0 à 10, de préférence 0 à 3,
Q représente hydroxy, amino, NHR₇, NR₇R₈, un résidu d'acide aminé ou un résidu peptide,
R₇ et R₈ représentent indépendamment l'un de l'autre alkyle en C₁ à C₁₈ ou hydroxyalkyle en C₁ à C₁₈,
et {POLY} est décrit par la formule II 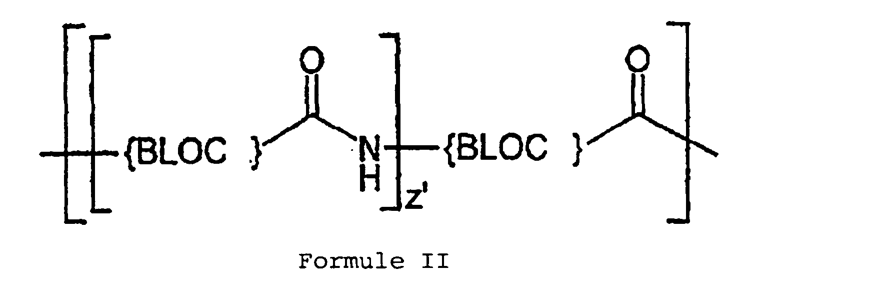 où {BLOC} est décrit, indépendamment l'un de l'autre, par la formule IIIA, 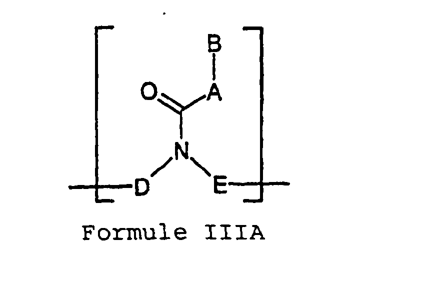 ou la formule IIIB 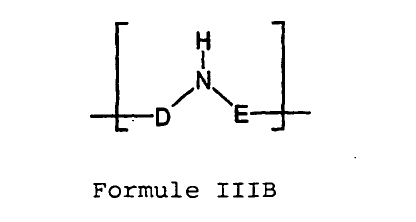 ou les formules IVA à IVG 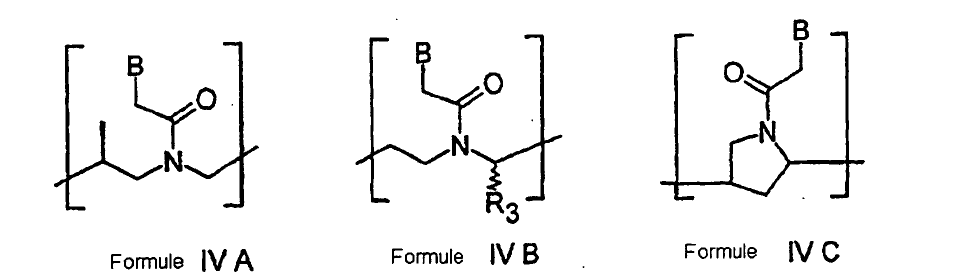 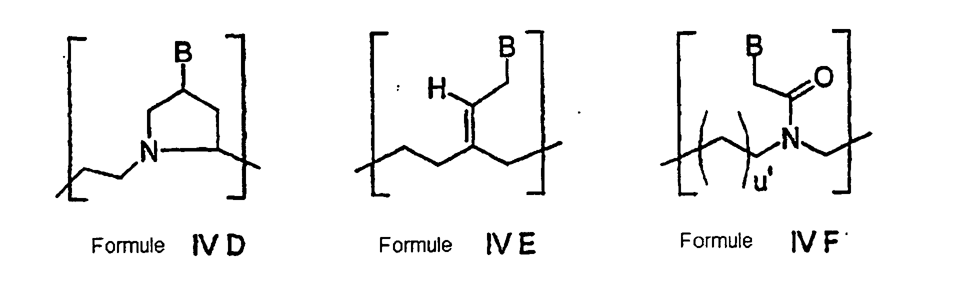 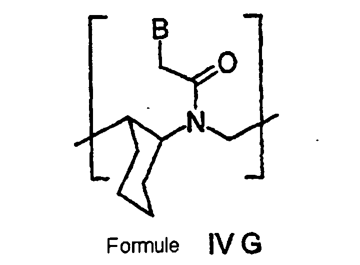 chaque élément (BLOC) pouvant être différent et où, en outre
z' vaut 0 à 100, de préférence 1 à 20, de manière particulièrement préférée 4 à 15,
A représente, indépendamment l'un de l'autre, un groupe (CR₁R₂)ₛ, où s représente 1 à 3, de préférence 1,
B représente, indépendamment l'un de l'autre, un radical aromatique, qui peut également présenter un caractère hétéroaromatique, ou hydrogène, hydroxy ou alkyle en C₁ à C₁₈,
ou une nucléobase existant à l'état naturel ou à l'état non naturel, usuelle dans la chimie des nucléotides ou sa forme précurseur de médicament,
au moins un radical B étant une nucléobase,
D représente, indépendamment l'un de l'autre, un groupe (CR₃R₄)ₜ, où t représente 2 à 10, de préférence 2 à 4, de manière particulièrement préférée 2, deux radicaux R₃ et R₄ adjacents pouvant également former un radical cycloalkyle en C₅ à C₈,
E représente, indépendamment l'un de l'autre, un groupe (CR₅R₆)_{u'}, deux radicaux R₅ et R₆ adjacents pouvant également former un radical cycloalkyle en C₅ à C₈ ou un composé spiro,
R₅ et R₆ représentent, indépendamment l'un de l'autre, un radical constitué par hydrogène ou alkyle en C₁ à C₆, de préférence hydrogène ou une chaîne latérale d'acide aminé,
ainsi que les sels physiologiquement acceptables du dérivé de PNA de formule I,
à condition qu'au moins un radical Y ou Z représente hydroxy, mercapto, oxyanion ou thioate.

2. Dérivé de PNA selon la revendication 1, où au moins un radical Y ou Z dans la formule I, dans une plage de pH de 4,5 à 14, de préférence de 6,5 à 12, de manière particulièrement préférée de 6,5 à 9, représente oxyanion ou thioate.

3. Dérivé de PNA selon l'une quelconque des revendications 1 à 2, où D représente (CH₂)₂.

4. Dérivé de PNA selon l'une quelconque des revendications 1 à 3, où A et E représentent CH₂.

5. Dérivé de PNA selon l'une quelconque des revendications 1 à 4, où Q représente un radical hydroxyaminoalkyle, de préférence un radical hydroxyaminohexyle, ou une séquence support, de préférence un Transportan, le facteur de croissance analogue à l'insuline, les signaux de localisation nucléaire ou un tag d'affinité, de préférence une chaîne (His)₆.

6. Dérivé de PNA selon l'une quelconque des revendications 1 à 5, où B représente adénine, cytosine, 5-méthylcytosine, guanine, thymine et uracile, ou il représente purine, 2,6-diaminopurine, N⁴N⁴-éthanocytosine, N⁶N⁶-éthano-2,6-diaminopurine, 5-(alcynyle en C₃ à C₆)uracile, 5-(alcynyle en C₃ à C₆)cytosine, 5-(1-propargylamino)uracile, 5-(1-propargylamino)cytosine, phénoxazine, 9-aminoéthoxyphénoxazine, 5-fluorouracile ou pseudoisocytosine, 5-(hydroxyméthyl)-uracile, 5-aminouracile, pseudouracile, dihydrouracile, 5-(alkyle en C₁ à C₆)uracile, 5-(alkyle en C₁ à C₆)cytosine, 5-(alcényle en C₂ à C₆)cytosine, 5-fluorocytosine, 5-chlorouracile, 5-chlorocytosine, 5-bromouracile, 5-bromocytosine, 7-désazaadénine, 7-désazaguanine, 8-azapurine, ou une purine substituée par 7-désaza-7-.

7. Dérivé de PNA selon l'une quelconque des revendications 1 à 6, où W représente oxygène et Y représente hydroxy ou oxyanion.

8. Dérivé de PNA selon l'une quelconque des revendications 1 à 7, où X représente un groupe U-((alcane en C₂ à C₂₂)diyl)-U, de préférence O-((alcane en C₂ à C₂₂)diyl)-O, de manière particulièrement préférée O-(CH₂)₂₋₆O, ou un groupe U-(CH₂CH₂-O)_{u'}, de préférence O-(CH₂CH₂-O)_{u'}, où u' représente de préférence 1 à 6.

9. Dérivé de PNA selon l'une quelconque des revendications 1 à 8, où Z représente phosphate ou un radical en C₁ à C₂₂ ou un radical en C₁ à C₂₂-U, de préférence un radical alcoxy en C₁ à C₂₂, de manière particulièrement préférée alcoxy en C₁₆, ou hydroxy-(C₁ à C₁₈)-U, de manière particulièrement préférée hydroxy- (C₁ à C₁₈)-O, de manière particulièrement préférée HO-(CH₂)₃₋₁₂O, ou un radical aminoalkyl-U, de préférence un radical aminoalcoxy, de manière particulièrement préférée 6-aminohexoxy ou 5-aminopentoxy, ou un groupe de formule R₉-(CH₂CH₂-O)ₘ, où R₉ représente de préférence OH ou NH₂ et m vaut 1 à 6, de manière particulièrement préférée HO(CH₂CH₂-O)₂, HO (CH₂CH₂-O)₆ et H₂N-(CH₂CH₂-O)₂, ou un radical mercaptoalkyl-U, de préférence un radical mercaptoalcoxy, de manière particulièrement préférée 6-mercaptohexyloxy.

10. Dérivé de PNA selon la revendication 1 à 7, où X et Z sont choisis indépendamment l'un de l'autre dans le groupe constitué par les dérivés de biotine, fluorescéine ou lexitropsine.

11. Dérivé de PNA selon l'une quelconque des revendications 1 à 7, où X et Z sont choisis, indépendamment l'un de l'autre, dans le groupe rhodamine, TAMRA ou colorant cyanine.

12. Dérivé de PNA selon l'une quelconque des revendications 1 à 7, où X et Z sont choisis, indépendamment l'un de l'autre, dans le groupe dabcyle, psoralène, acridine, DNP ou cholestérol.

13. Dérivé de PNA selon l'une quelconque des revendications 1 à 13, où la séquence de base est dirigée contre des parties de gènes suppresseurs de tumeur, d'oncogènes ou de télomérases ou leurs produits de transcription.

14. Dérivé de PNA selon la revendication 13, où la séquence de base de la partie de PNA est dirigée contre le début de la translation de HA-ras ARNm.

15. Dérivé de PNA selon l'une quelconque des revendications 1 à 14 destiné à une utilisation comme médicament.

16. Utilisation d'un dérivé de PNA selon l'une quelconque des revendications 1 à 14 pour la préparation d'un médicament destiné à la thérapie tumorale.

17. Dérivé de PNA selon l'une quelconque des revendications 1 à 14 destiné à une utilisation comme agent de diagnostic.

18. Utilisation d'un dérivé de PNA selon l'une quelconque des revendications 1 à 14 pour la détection de microorganismes et/ou de virus in vitro.

19. Utilisation d'un dérivé de PNA selon l'une quelconque des revendications 1 à 14 pour la détection et/ou la quantification d'acides nucléiques in vitro.

20. Utilisation d'un dérivé de PNA selon l'une quelconque des revendications 1 à 14 comme réactif de détection pour l'hybridation in situ ou la fluorescence-in-situ-hybridation in vitro.

21. Utilisation d'un dérivé de PNA selon l'une quelconque des revendications 1 à 14 comme agent antisens, anti-gène, Decoy ou chiméraplaste in vitro.

22. Réactif de détection contenant un dérivé de PNA selon l'une quelconque des revendications 1 à 14.

23. Puce à PNA, contenant un dérivé de PNA selon l'une quelconque des revendications 1 à 14.

24. Biosonde contenant un dérivé de PNA selon l'une quelconque des revendications 1 à 14.

25. Médicament, contenant un dérivé de PNA selon l'une quelconque des revendications 1 à 14 et le cas échéant d'autres additifs et/ou substances support pharmacologiquement acceptables.

26. Agent antisens, contenant un dérivé de PNA selon l'une quelconque des revendications 1 à 14.

27. Procédé pour la préparation d'un dérivé de PNA selon l'une quelconque des revendications 1 à 14, où
a) la structure de base du PNA est élaborée partant de la terminaison C au moyen d'acides amidonucléiques actifs, et
b) on transforme avec un réactif de phosphorylation au niveau de la terminaison N.

28. Procédé selon la revendication 27, où le PNA est préparé avec utilisation des groupes de protection t-butyloxycarbonyle (BOC), 9-fluorénylméthoxycarbonyle (Fmoc) ou monométhoxytrityle (Mmt).

29. Procédé selon la revendication 28, où le PNA est préparé avec utilisation de supports solides.

30. Procédé selon la revendication 29, où on utilise comme support solide du CPG, du Tentagel ou de l'aminométhylpolystyrène.

31. Procédé pour la préparation d'un médicament où
a) on prépare un dérivé de PNA selon l'une quelconque des revendications 27 à 30 et
b) on le mélange le cas échéant avec d'autres additifs et/ou substances support pharmacologiquement acceptables.

32. Procédé pour la préparation d'une puce à PNA, où, selon l'une quelconque des revendications 27 à 30, on prépare d'abord un dérivé de PNA puis on le fixe sur un support solide ou on prépare le dérivé de PNA directement sur le support.

33. Procédé pour la préparation d'un dérivé de PNA de Formule I, selon la revendication 27 à 30, **caractérisé en outre en ce que** le PNA est purifié en exploitant le caractère acide du radical phosphoré par chromatographie ou électrophorèse.

34. Procédé selon la revendication 33, où on purifie un dérivé de PNA par chromatographie au moyen d'une phase stationnaire basique et d'un éluant acide ou contenant un sel.

35. Procédé selon la revendication 34, où la phase stationnaire est un échangeur d'anions ou une phase en mode mixte.


### SEQUENZPROTOKOLL

<110> Aventis Pharma Deutschland GmbH
<120> Polyamidnukleinsäure-Derivate, Mittel und Verfahren zu ihrer Herstellung
<130> D-2000/A022
<140> 10019136.3-43
   <141> 2000-04-18
<160> 64
<170> PatentIn Ver. 2.1
<210> 1
   <211> 21
   <212> DNA
   <213> Künstliche Sequenz
<220>
   <221> misc_binding
   <222> (1)..(21)
<220>
   <223> Beschreibung der künstlichen Sequenz: Oligonukleotide
<400> 1
   gcgtttgctc ttcttcttgc g 21
<210> 2
   <211> 15
   <212> DNA
   <213> Künstliche Sequenz
<220>
   <221> misc_binding
   <222> (1)..(15)
<220>
   <223> Beschreibung der künstlichen Sequenz: Oligonukleotide
<400> 2
   aacgttgagg ggcat 15
<210> 3
   <211> 18
   <212> DNA
   <213> Künstliche Sequenz
<220>
   <223> Beschreibung der künstlichen Sequenz: Oligonukleotide
<220>
   <221> misc_binding
   <222> (1)..(18)
<400> 3
   gtgccggggt cttcgggc 18
<210> 4
   <211> 17
   <212> DNA
   <213> Künstliche Sequenz
<220>
   <223> Beschreibung der künstlichen Sequenz: Oligonukleotide
<220>
   <221> misc_binding
   <222> (1)..(17)
<400> 4
   cgagaacatc atcgtgg 17
<210> 5
   <211> 21
   <212> DNA
   <213> Künstliche Sequenz
<220>
   <223> Beschreibung der künstlichen Sequenz: Oligonukleotide
<220>
   <221> misc_binding
   <222> (1)..(21)
<400> 5
   ggagaacatc atggtcgaaa g 21
<210> 6
   <211> 21
   <212> DNA
   <213> Künstliche Sequenz
<220>
   <223> Beschreibung der künstlichen Sequenz: Oligonukleotide
<220>
   <221> misc_binding
   <222> (1)..(21)
<400> 6
   ggagaacatc atggtcgaaa g 21
<210> 7
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 7
   ggggaaagcc cggcaagggg 20
<210> 8
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 8
   cacccgcctt ggcctcccac 20
<210> 9
   <211> 18
   <212> DNA
   <213> Künstliche Sequenz
<220>
   <223> Beschreibung der künstlichen Sequenz: Oligonukleotide
<220>
   <221> misc_binding
   <222> (1)..(18)
<400> 9
   gggactccgg cgcagcgc 18
<210> 10
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 10
   ggcaaacttt cttttcctcc 20
<210> 11
   <211> 19
   <212> DNA
   <213> Künstliche Sequenz
<220>
   <223> Beschreibung der künstlichen Sequenz: Oligonukleotide
<220>
   <221> misc_binding
   <222> (1)..(19)
<400> 11
   gggaaggagg aggatgagg 19
<210> 12
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 12
   acacccaatt ctgaaaatgg 20
<210> 13
   <211> 21
   <212> DNA
   <213> Künstliche Sequenz
<220>
   <223> Beschreibung der künstlichen Sequenz: Oligonukleotide
<220>
   <221> misc_binding
   <222> (1)..(21)
<400> 13
   ggcagtcatc cagcttcgga g 21
<210> 14
   <211> 18
   <212> DNA
   <213> Künstliche Sequenz
<220>
   <223> Beschreibung der künstlichen Sequenz: Oligonukleotide
<220>
   <221> misc_binding
   <222> (1)..(18)
<400> 14
   tctcccagcg tgcgccat 18
<210> 15
   <211> 19
   <212> DNA
   <213> Künstliche Sequenz
<220>
   <223> Beschreibung der künstlichen Sequenz: Oligonukleotide
<220>
   <221> misc_binding
   <222> (1)..(19)
<400> 15
   gcgctgatag acatccatg 19
<210> 16
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 16
   ggaggcccga cc 12
<210> 17
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 17
   ggtttcggag gc 12
<210> 18
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 18
   tggtggaggt ag 12
<210> 19
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 19
   gcatggtgga gg 12
<210> 20
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 20
   ttggcatggt gg 12
<210> 21
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 21
   gcctgggacc ac 12
<210> 22
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 22
   cagcctggga cc 12
<210> 23
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 23
   aggtccctgt tcgggcgcca 20
<210> 24
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 24
   tgcagcctgg ga 12
<210> 25
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 25
   gtgcagcctg gg 12
<210> 26
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 26
   ggtgcagcct gg 12
<210> 27
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 27
   atgggtgcag cc 12
<210> 28
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 28
   ggcttgaaga tg 12
<210> 29
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 29
   gcagcccccg ca 12
<210> 30
   <211> 12
   <212> DNA
   <213> Künstliche Sequenz
<220>
   <223> Beschreibung der künstlichen Sequenz: Oligonukleotide
<220>
   <221> misc_binding
   <222> (1)..(12)
<400> 30
   gcagcagccc cc                                                    12
<210> 31
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 31
   tcccgcctgt gacatgcatt                                            20
<210> 32
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 32
   gttctcgctg gtgagtttca                                            20
<210> 33
   <211> 18
   <212> DNA
   <213> Künstliche Sequenz
<220>
   <223> Beschreibung der künstlichen Sequenz:
   Oligonukleotide
<220>
   <221> misc_binding
   <222> (1)..(18)
<400> 33
   gcgtgcctcc tcactggc 18
<210> 34
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 34
   gcggggctcc atgggggtcg 20
<210> 35
   <211> 18
   <212> DNA
   <213> Künstliche Sequenz
<220>
   <223> Beschreibung der künstlichen Sequenz: Oligonukleotide
<220>
   <221> misc_binding
   <222> (1)..(18)
<400> 35
   gcagtaagca tccatatc 18
<210> 36
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 36
   gcccaagctg gcatccgtca 20
<210> 37
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 37
   cccccaccac ttcccctctc 20
<210> 38
   <211> 20
   <212> DNA
   <213> Künstliche Sequenz
<220>
   <223> Beschreibung der künstlichen Sequenz: Oligonukleotide
<220>
   <221> misc_binding
   <222> (1)..(20)
<400> 38
   ctcccccacc acttcccctc 20
<210> 39
   <211> 19
   <212> DNA
   <213> Künstliche Sequenz
<220>
   <223> Beschreibung der künstlichen Sequenz: Oligonukleotide
<220>
   <221> misc_binding
   <222> (1)..(19)
<400> 39
   gctgggagcc atagcgagg 19
<210> 40
   <211> 21
   <212> DNA
   <213> Künstliche Sequenz
<220>
   <223> Beschreibung der künstlichen Sequenz: Oligonukleotide
<220>
   <221> misc_binding
   <222> (1)..(21)
<400> 40
   actgctgcct cttgtctcag g 21
<210> 41
   <211> 22
   <212> DNA
   <213> Künstliche Sequenz
<220>
   <223> Beschreibung der künstlichen Sequenz: Oligonukleotide
<220>
   <221> misc_binding
   <222> (1)..(22)
<400> 41
   caatcaatga cttcaagagt tc 22
<210> 42
   <211> 18
   <212> DNA
   <213> Künstliche Sequenz
<220>
   <223> Beschreibung der künstlichen Sequenz:
   Oligonukleotide
<220>
   <221> misc_binding
   <222> (1)..(18)
<400> 42
   gcggcggaaa agccatcg 18
<210> 43
   <211> 18
   <212> DNA
   <213> Künstliche Sequenz
<220>
   <223> Beschreibung der künstlichen Sequenz: Oligonukleotide
<220>
   <221> misc_binding
   <222> (1)..(18)
<400> 43
   gtgtcggggt ctccgggc 18
<210> 44
   <211> 15
   <212> DNA
   <213> Künstliche Sequenz
<220>
   <223> Beschreibung der künstlichen Sequenz: Oligonukleotide
<220>
   <221> misc_binding
   <222> (1)..(15)
<400> 44
   cacgttgagg ggcat 15
<210> 45
   <211> 15
   <212> DNA
   <213> Künstliche Sequenz
<220>
   <223> Beschreibung der künstlichen Sequenz: Oligonukleotide
<220>
   <221> misc_binding
   <222> (1)..(15)
<400> 45
   cagctgcaac ccagc 15
<210> 46
   <211> 18
   <212> DNA
   <213> Künstliche Sequenz
<220>
   <223> Beschreibung der künstlichen Sequenz: Oligonukleotide
<220>
   <221> misc_binding
   <222> (1)..(18)
<400> 46
   gtcttccata gttactca 18
<210> 47
   <211> 18
   <212> DNA
   <213> Künstliche Sequenz
<220>
   <223> Beschreibung der künstlichen Sequenz: Oligonukleotide
<220>
   <221> misc_binding
   <222> (1)..(18)
<400> 47
   gatcaggcgt gcctcaaa                                                18
<210> 48